# FASCICULE DE BREVET EUROPEEN

(11) **EP 1 218 510 B1**
(45) Date de publication et mention de la délivrance du brevet: **11.11.2015**
(21) Numéro de dépôt: 00964322.2
(22) Date de dépôt: 19.09.2000
(51) Int. Cl.: C07K 14/415, C12N 15/82

(54) **PROMOTEURS SPECIFIQUES DE L'ALBUMEN DES GRAINES DE VEGETAUX**
PROMOTOREN, DIE IN DER EIWEISS-REGION DES PFLANZENSAMENS AKTIV SIND
PLANT SEED ENDOSPERM-SPECIFIC PROMOTER

(30) Priorité: 01.10.1999 FR 9912305
(43) Date de publication de la demande: 03.07.2002
(73) Titulaire: Biogemma, 75001 Paris (FR); CENTRE NATIONAL DE LA RECHERCHE SCIENTIFIQUE -CNRS-, 75794 Paris Cedex 16 (FR)
(72) Inventeur: BONELLO, Jean-François, F-77600 Bussy St Georges (FR); ROGOWSKY, Peter, F-69007 Lyon (FR); PEREZ, Pascual, F-63450 Chanonat (FR)
(74) Mandataire: Jacobson, Claude
(86) Numéro de dépôt international: PCT/FR2000/002596
(87) Numéro de publication internationale: WO 2001/025439

(56) Documents cités:
- EP-A- 0 412 006
- WO-A-91/09957
- WO-A-93/09237
- WO-A-97/28247
- WO-A-98/08961
- WO-A-98/10062
- WO-A-98/26064
- WO-A-99/40209
- OPSAHL-FERSTAD, H.-G., ET AL.: "ZmEsr, a novel endosperm-specific gene expressed in a restricted region around the maize embryo." THE PLANT JOURNAL, vol. 12, no. 1, 1997, pages 235-246, XP002140059 & DATABASE EMBL [en ligne] ACCESSION NO:X98497, 20 août 1997 (1997-08-20) & DATABASE EMBL [en ligne] ACCESSSION NO:X98499, 20 août 1997 (1997-08-20) & DATABASE EMBL [en ligne] ACCESSION NO:X99968, 20 août 1997 (1997-08-20) & DATABASE EMBL [en ligne] ACCESSION NO:X99969, 20 août 1997 (1997-08-20) & DATABASE EMBL [en ligne] ACCESSION NO:X99970, 20 août 1997 (1997-08-20)
- BONELLO, J.-F., ET AL.: "Esr genes show different levels of expression in the same region of maize endosperm" GENE, vol. 246, 2000, pages 219-227, XP002140061 -& DATABASE EMBL [en ligne] ACCESSION NO:AJ251318, 26 avril 2000 (2000-04-26) ROGOWSKY, P.M.: " Zea mays Esr1 gene, 5' flanking region" XP002157139 -& DATABASE EMBL [en ligne] ACCESSION NO:AJ251319, 26 avril 2000 (2000-04-26) ROGOWSKY, P.M.: "Zea mays Esr3 gene, 5' flanking region" XP002157140 -& DATABASE EMBL [en ligne] ACCESSION NO:AJ251320, 26 avril 2000 (2000-04-26) ROGOWSKY, P.M.: " Zea mays Esr2 gene, 5' flanking region" XP002157141
- BEATTY, M.K., ET AL.: "Zea mays SU1 isoamylase (sugary1) gene, complete cds" EMBL ACCESSION NO:AF030882, 17 novembre 1997 (1997-11-17), XP002140060
- CZAKO M ET AL: "DIFFERENTIAL MANIFESTATION OF SEED MORTALITY INDUCED BY SEED-SPECIFIC EXPRESSION OF THE GENE FOR DIPHTHERIA TOXIN A CHAIN IN ARABIDOPSIS AND TOBACCO" MOLECULAR AND GENERAL GENETICS,DE,SPRINGER VERLAG, BERLIN, vol. 235, 1 janvier 1992 (1992-01-01), pages 33-40, XP002038059 ISSN: 0026-8925
- DATABASE BIOSIS [en ligne] BIOSCIENCES INFORMATION SERVICE, PHILADELPHIA, PA, US; 1998 MA QING-HU ET AL: "Seed-specific expression of the isopentenyl transferase gene (ipt) in transgenic tobacco." Database accession no. PREV199800228383 XP002140062 & AUSTRALIAN JOURNAL OF PLANT PHYSIOLOGY 1998, vol. 25, no. 1, 1998, pages 53-59, ISSN: 0310-7841
- Z ZHENG ET AL: "5' distal and proximal cis-acting regulator elements are required for developmental control of a rice seed storage protein glutelin gene.", PLANT JOURNAL, vol. 4, no. 2, 1 August 2013 (2013-08-01), pages 357-366, XP055066695, DOI: 10.1046/j.1365-313X.1993.04020357.x

## Description

La présente invention se rapporte au contrôle de l'expression des gènes au cours du développement de l'albumen. Elle concerne en particulier des séquences nucléotidiques promotrices permettant une expression à la fois spécifique à l'interface entre l'embryon et l'albumen et précoce au cours du développement de l'albumen.

L'albumen, formation caractéristique de la graine des Angiospermes, est un tissu nourricier pour l'embryon. C'est un tissu complexe dans sa structure et son développement, en particulier chez les céréales. La zone centrale de l'albumen consiste en de larges cellules à vacuoles, qui stockent les réserves d'amidon et de protéines, tandis que la région entourant l'embryon se distingue par des cellules plutôt petites, occupées en grande partie par du cytoplasme. On ne connaît pas à ce jour la fonction de ces cellules appelées "cellules cytoplasmiques denses" (Schel et al, 1984). En 1997, Opsahl et al ont identifié un gène exprimé spécifiquement dans cette région restreinte autour de l'embryon de maïs, gène qu'ils ont dénommé *Esr* (pour « Embryo Surrounding Region »).

La demande WO 98/10062 décrit l'utilisation d'un promoteur du gène de la glutéline-1 de riz pour permettre l'expression spécifique d'un gène d'origine non végétale dans l'albumen des grains de monocotylédones.

L'article Zheng et al. (1993, The Plant Journal, 357-366) divulgue les éléments régulateurs requis pour le contrôle développemental du gène de la *glutéline* des semences de riz. Il est précisé qu'un élément régulateur de la région distale -5,1 à -1,8 kb permet une augmentation de l'expression de la glutéline au cours du développement de l'endosperme.

Les auteurs de la présente invention ont maintenant isolé des séquences nucléotidiques promotrices permettant une expression des séquences codantes auxquelles elles peuvent être liées, qui est spécifique de la région de l'albumen entourant l'embryon dans les graines des Angiospermes et qui intervient en particulier dans les stades précoces du développement de l'albumen.

De telles séquences promotrices sont particulièrement utiles pour cibler ou réguler l'expression de gènes d'intérêt.

Dans le cadre d'une amélioration des plantes par transgénèse, on peut lier de manière opérante une telle séquence nucléotidique promotrice à une séquence codant pour un gène d'intérêt.

La construction nucléotidique, préférentiellement insérée dans un vecteur, peut être utilisée pour transformer des cellules végétales de manière stable, de telle sorte que la plante ainsi transformée contienne dans son génome le gène d'intérêt associé à la séquence promotrice de l'invention.

Les graines qui se développent, par fécondation, à partir de cette plante, contiennent également dans leur génome ce transgène.

Du fait de son association à la séquence promotrice de l'invention, ce transgène d'intérêt ne s'exprimera que dans la région de l'albumen entourant l'embryon, c'est-à-dire dans les cellules cytoplasmiques denses telles que mentionnées précédemment.

L'expression du transgène débute dès les premiers jours après la pollinisation, plus précisément dès le quatrième jour après la pollinisation.

La demande décrit des séquences promotrices choisies parmi les séquences comprenant les séquences SEQ ID n° 1, n°2, n°3, n°4, n°5, n° 6, ou n° 7 ou toute séquence nucléotidique homologue de celles-ci.

La séquence SEQ ID n° 1 correspond au promoteur du gène *Esr1.*

La séquence SEQ ID n° 2 correspond au promoteur du gène *Esr2.*

La séquence SEQ ID n° 3 correspond au promoteur du gène *Esr3.*

La séquence SEQ ID n° 4 correspond au promoteur du gène *Esr4.*

La séquence SEQ ID n° 5 correspond à un fragment de 499 paires de bases sur SEQ ID n° 2 (nucléotides 1995-2493).

La séquence SEQ ID n° 6 correspond à un fragment de 507 paires de bases sur SEQ ID n° 3 (nucléotides 1202-1708).

La séquence SEQ ID n° 7 est une séquence consensus de 265 nucléotides, obtenue par comparaison entre les séquences SEQ ID n°1, n°2 et n°3.

Par "séquence nucléotidique homologue", on entend toute séquence nucléotidique qui diffère de la séquence SEQ ID n°1, n° 2, n° 3 n° 4 , n°5, n°6 ou n° 7, par substitution, délétion, et/ou insertion d'un ou plusieurs nucléotides, à des positions telles que ces séquences nucléotidiques homologues conservent la propriété de promoteur spécifique des séquences SEQ ID n° 1 à n° 7.

De préférence, une telle séquence nucléotidique homologue est identique à au moins 70 % des séquences SEQ ID n° 1 à n° 7, de préférence au moins 80 %, de préférence encore au moins 95 %.

L'invention concerne une séquence nucléotidique promotrice isolée permettant une expression des séquences codantes auxquelles elle est liée, ladite expression étant i) spécifique de la région de l'albumen entourant l'embryon dans les graines des Angiospermes et ii) intervenant en particulier dans les stades précoces du développement de l'albumen, consistant en SEQ ID N° 7 ou une séquence identique à au moins 80 % de SEQ ID N° 7.

L'homologie est généralement déterminée en utilisant un logiciel d'analyse de séquences (par exemple, Sequence Analysis Software Package of the Genetics Computer Group, University of Wisconsin Biotechnology Center, 1710 University Avenue, Madison, WI 53705). Des séquences nucléotidiques similaires sont alignées pour obtenir le maximum de degré d'homologie (i.e. identité). A cette fin, il peut être nécessaire d'introduire de manière artificielle des espaces (« gaps ») dans la séquence. Une fois l'alignement optimal réalisé, le degré d'homologie (i.e. identité) est établi par enregistrement de toutes les positions pour lesquelles les nucléotides des deux séquences comparées sont identiques, par rapport au nombre total de positions.

De manière préférentielle, une telle séquence nucléotidique homologue hybride spécifiquement aux séquences complémentaires des séquences SEQ ID n° 1 à n° 7; dans des conditions stringentes. Les paramètres définissant les conditions de stringence dépendent de la température à laquelle 50% des brins appariés se séparent (Tm).

Pour les séquences comprenant plus de 30 bases, Tm est définie par la relation : Tm=81,5+0,41(%G+C)+16,6Log(concentration en cations)-0,63(%formamide) -(600/nombre de bases) (Sambrook et al, Molecular Cloning, A laboratory manual, Cold Spring Harbor laboratory Press, 1989, pages 9.54-9.62).

Pour les séquences de longueur inférieure à 30 bases, Tm est définie par la relation : Tm= 4(G+C) + 2 (A+T).

Dans des conditions de stringence appropriées, auxquelles les séquences aspécifiques n'hybrident pas, la température d'hybridation est approximativement de 5 à 30°C, de préférence de 5 à 10°C en dessous de Tm, et les tampons d'hybridation utilisés sont de préférence des solutions de force ionique élevée telle qu'une solution 6xSSC par exemple.

Les différentes séquences nucléotidiques de l'invention peuvent être d'origine artificielle ou non. Il peut s'agir de séquences d'ADN, obtenues par criblage de banques de séquences au moyen de sondes élaborées sur la base des séquences SEQ ID n° 1 à n° 7. De telles banques peuvent être préparées par des techniques classiques de biologie moléculaire, connues de l'homme de l'art.

Les séquences nucléotidiques selon l'invention peuvent également être préparées par synthèse chimique, ou encore par des méthodes mixtes incluant la modification chimique ou enzymatique de séquences obtenues par criblage des banques.

Les séquences nucléotidiques promotrices de l'invention sont de préférence des séquences isolées à partir de céréales, en particulier de maïs.

Les séquences nucléotidiques promotrices selon la présente invention peuvent notamment être isolées par des méthodes de PCR inversée ou de marche sur le génome (Devic et al , 1997).

Les séquences nucléotidiques promotrices de l'invention peuvent en outre comprendre ou être associées à un motif régulateur en *cis* CTACACCA, de préférence répété en tandem, ou tout autre motif comprenant une ou plusieurs bases dégénérées ayant la même fonction.

La présente invention a également pour objet une construction nucléotidique, appelée cassette d'expression, comprenant une séquence nucléotidique promotrice selon l'invention telle que définie précédemment liée de manière opérante à au moins un gène d'intérêt.

Ledit gène d'intérêt peut être également associé à d'autres éléments de régulation tels que des activateurs et des séquences de terminaison de transcription (terminateurs). A titre d'exemple de terminateur utilisable dans de telles constructions, on peut citer l'extrémité 3' du gène de la nopaline synthase d'*Agrobacterium tumefaciens.*

Ledit gène d'intérêt peut par exemple coder pour une protéine impliquée dans le développement de l'embryon et/ou de l'albumen, la croissance cellulaire, le métabolisme des sucres (invertase) et acides gras, le flux de nutriments (transporteurs). Il peut également coder pour une protéine toxique, ou encore pour une protéine activatrice ou inhibitrice d'autres gènes, telle qu'une protéine inhibant un facteur de transcription (domaines de répression de type engrailed (Poole et al, 1985) ou corépresseurs par exemple).

Selon un mode préféré, le gène d'intérêt code pour une protéine dont l'expression spécifique dans la zone entourant l'embryon permettra de jouer sur la taille de l'embryon et/ou son développement. A titre d'exemple, ce gène peut coder pour une barnase ou isopentényl-transferase.

Le gène d'intérêt peut être placé en orientation sens ou antisens.

La séquence nucléotidique promotrice de l'invention peut également être associée à un gène marqueur, par exemple un gène permettant de sélectionner une plante transformée d'une plante qui ne contient pas l'ADN étranger transfecté. Comme gène marqueur, on peut citer notamment un gène conférant une résistance à un antibiotique (Herrera-Estrella et al, EMBO J. 2, 987-995 (1983) ou une résistance à un herbicide (EP 242 246).

L'invention a également pour objet tout vecteur nucléotidique, tel qu'un plasmide, utilisable pour la transformation de cellules hôtes, caractérisé en ce qu'il comprend une cassette d'expression telle que définie précédemment. La construction de vecteurs d'expression pour la transformation est à la portée de l'homme du métier suivant les techniques standards.

L'invention a également pour objet une cellule hôte transformée de plante Angiosperme, notamment de céréale, comprenant un vecteur selon l'invention.

L'invention concerne en outre une plante transgénique ou partie de plante transgénique, notamment semence, fruit et pollen, générée à partir d'une telle cellule.

Parmi les cellules susceptibles d'être transformées selon le procédé de l'invention, on peut citer à titre d'exemples des cellules de plantes de grandes cultures (maïs, blé, colza, tournesol, pois, soja, orge..) ou des plantes potagères et fleurs. Préférentiellement, on peut choisir des plantes connues pour contenir de grandes réserves (protéiques, glucidiques et lipidiques), notamment les plantes céréalières ou les plantes oléagineuses.

Les plantes hybrides obtenues par le croisement de plantes selon l'invention et contenant dans leur génome une cassette d'expression selon l'invention, font aussi partie de l'invention.

L'invention a également pour objet un procédé d'obtention d'une plante Angiosperme présentant des qualités agronomiques ou nutritionnelles améliorées, comprenant les étapes consistant à :
- transformer au moins une cellule de plante Angiosperme par un vecteur tel que défini précédemment ;
- cultiver la cellule ainsi transformée de manière à générer une plante contenant dans son génome une cassette d'expression selon l'invention.

La transformation de cellules végétales peut être réalisée par les techniques connues de l'homme du métier.

On peut citer notamment les méthodes de transfert direct de gènes telles que la microinjection directe dans des embryoïdes de plante (Neuhaus et Coll., 1987), l'infiltration sous vide (Bechtold et al., 1993) ou l'électroporation (Chupeau et Coll., 1989) ou encore la précipitation directe au moyen de PEG (Schocher et Coll., 1986) ou le bombardement par canon de particules recouvertes de l'ADN plasmidique d'intérêt (Fromm M. et al., 1990).

On peut également infecter la plante par une souche bactérienne notamment d'Agrobacterium. Selon un mode de réalisation du procédé de l'invention, les cellules végétales sont transformées par un vecteur selon l'invention, ledit hôte cellulaire étant susceptible d'infecter lesdites cellules végétales en permettant l'intégration dans le génome de ces dernières, des séquences nucléotidiques d'intérêt initialement contenues dans le génome de vecteur susmentionné. Avantageusement, l'hôte cellulaire susmentionné utilisé est *Agrobacterium tumefaciens,* notamment selon la méthode décrite dans l'article d'An et al, (1986), ou encore *Agrobacterium rhizogenes,* notamment selon la méthode décrite dans l'article de Guerche et al, (1987).

Par exemple, la transformation des cellules végétales peut être réalisée par le transfert de la région T du plasmide circulaire extra-chromosomique inducteur de tumeurs Ti d'*Agrobacterium tumefaciens,* en utilisant un système binaire (Watson et al, 1994). Pour ce faire, deux vecteurs sont construits. Dans l'un de ces vecteurs, la région T a été éliminée par délétion, à l'exception des bordures droite et gauche, un gène marqueur étant inséré entre eux pour permettre la sélection dans les cellules de plantes. L'autre partenaire du système binaire est un plasmide Ti auxiliaire, plasmide modifié qui n'a plus de région T mais contient toujours les gènes de virulence *vir,* nécessaires à la transformation de la cellule végétale.

Selon un mode préféré, on peut utiliser la méthode décrite par Ishida et al (1996) pour la transformation des Monocotylédones.

Selon un autre protocole, la transformation est réalisée selon la méthode décrite par Finer et al, (1992) utilisant le canon à particules de tungstène ou d'or.

L'invention a également pour objet l'utilisation des séquences nucléotidiques promotrices selon l'invention, dans des constructions moléculaires destinées à améliorer la qualité agronomique, alimentaire, ou industrielle d'une plante, en jouant notamment sur la taille de l'embryon ou de l'albumen et/ou son développement.

En effet, une action précoce et spécifique sur le développement des tissus de l'embryon et de l'albumen peut être recherchée : selon la taille relative de l'un ou l'autre tissu, il sera possible d'obtenir des graines ou fruits enrichis en amidon (gros albumen) et/ou huile (gros embryon), via l'utilisation, respectivement de gènes stimulateurs (hormone du cycle cellulaire par exemple) ou de gènes inhibiteurs (protéine toxique ou inhibiteur de transcription, par exemple). Des albumens sans embryons pourraient également être obtenus selon ce modèle, pour des applications industrielles en amidonnerie et semoulerie.

A titre d'exemple, l'utilisation de gènes codant pour des hormones (cytokinines, auxines) du cycle cellulaire, sous contrôle des promoteurs décrits selon l'invention, permettrait de modifier les processus de cellularisation et, de façon corrélée, le développement de l'albumen, au vu des travaux de R. J. Scott (1998).

Une action sur l'accumulation de nutriments dans l'embryon et de l'albumen peut être également recherchée, en utilisant par exemple comme gènes d'intérêts, des gènes codant pour des transporteurs de nutriments (sucres notamment) aux interfaces plante mère/albumen et albumen/embryon, ou des gènes codant pour des inhibiteurs de ces transports, pour une accumulation différentielle de nutriments dans l'albumen ou l'embryon.

L'invention vise donc également des procédés pour modifier les qualités agronomiques et/ou nutritionnelles d'une plante, par une action ciblée et précoce sur le développement de l'embryon/albumen, utilisant la transformation des plantes avec un vecteur selon l'invention. En particulier, elle s'intéresse à la modification de la taille et/ou du développement de l'embryon/l'albumen. Elle vise également l'altération du développement de l'embryon, en vue de produire des grains sans embryons pour les céréales notamment, présentant un intérêt pour les industries de l'amidonnerie et de la semoulerie.

L'invention a plus précisément pour objet l'utilisation d'une cassette d'expression telle que définie précédemment, pour l'obtention d'une plante Angiosperme transgénique présentant des qualités agronomiques ou nutritionnelles améliorées.

De manière avantageuse, la plante transgénique obtenue peut produire des graines à teneurs en amidon ou en huile modifiées en comparaison avec une plante non transformée.

L'invention concerne également l'utilisation des plantes transgéniques obtenues selon l'invention, ou parties de ces plantes, notamment semences, grains et fruits pour la préparation de produits dérivés, notamment de produits alimentaires.

Rentrent également dans l'invention les produits obtenus, que ce soit des semences enrichies en huile, farines de semences ou grains enrichis en amidon ou en huile.

L'invention a enfin pour objet toute composition pour l'alimentation humaine ou animale préparée à partir desdits produits obtenus.

Les figures et exemples ci-après illustrent l'invention sans en limiter sa portée.

### LEGENDE DES FIGURES

- La figure 1 représente un schéma illustrant les étapes du clonage des promoteurs *Esr*.
- La figure 2 représente la carte de restriction du plasmide L82/34, comprenant notamment le promoteur *pEsr1* fusionné à *Gus.*
- La figure 3 représente la carte de restriction du plasmide L124/19, comprenant notamment le promoteur *pEsr2* fusionné à *Gus.*
- La figure 4 représente la carte de restriction du plasmide L127a5, comprenant notamment le promoteur *pEsr3* fusionné à *Gus.*
- La figure 5 représente la carte de restriction du plasmide L78a1, comprenant notamment le promoteur *pEsr1* fusionné à *Ipt.*
- La figure 6 représente la carte de restriction du plasmide L125a2, comprenant notamment le promoteur *pEsr2* fusionné à *Ipt.*
- La figure 7 représente la carte de restriction du plasmide L77a101, comprenant notamment le promoteur *pEsr1* fusionné à *Barnase.*

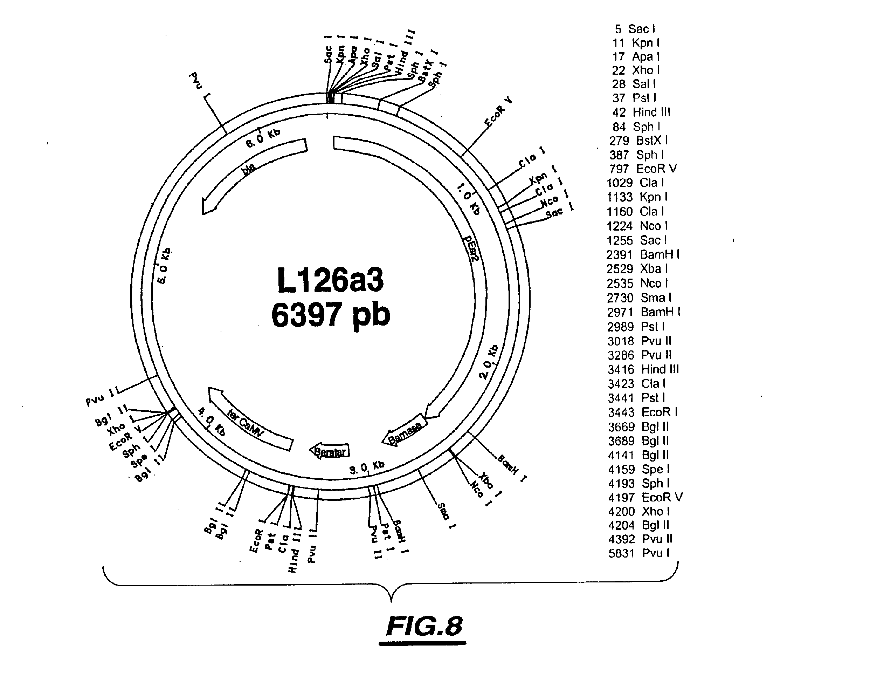

- La figure 8 représente la carte de restriction du plasmide L126a3, comprenant notamment le promoteur *pEsr2* fusionné à *Barnase.*
- La figure 9 représente une comparaison des séquences des promoteurs des gènes *Esr1, Esr2* et *Esr3* (respectivement prEsr1, prEsr2 et prEsr3, ou SEQ ID n° 1, SEQ ID n° 2 et SEQ ID n° 3), les parties conservées étant alignées.
- La figure 10 représente la carte de restriction du plasmide pWP280.
- La figure 11 représente la carte de restriction du plasmide L129/46, comprenant notamment le promoteur *pEsr2* fusionné à la séquence *Esr2* en antisens.

### EXEMPLES

### EXEMPLE 1 :

### Expression quantitative Esr1, 2, 3

Les travaux de Opsahl-Ferstad et al. (1997) ont permis d'identifier par "differential display" un amplicon spécifique de l'albumen *Esrₐ1* (numéro d'accès sur la base de données EMBL : X98495) et d'isoler, par criblage de banques génomiques et complémentaires sur lignée hybride HD5*HD7 (Barloy et coll., 1989) et lignée A188 (Gerdes et Tracy, 1993) respectivement, des clones correspondants. A partir des séquences génomiques *Esr1g1* (numéro d'accès sur EMBL : X98497) et *Esr2g1* (numéro d'accès sur EMBL : X98499) et Esr3g2 (numéro d'accès sur EMBL : X99970) notamment, trois gènes *Esr1, Esr2* et *Esr3* ont pu être mis en évidence.

Les auteurs de la présente invention ont évalué les contributions relatives d'expression de chacun des gènes Esr grâce à des expériences de RT-PCR, digestion par enzymes de restriction et quantification selon les méthodes connues de l'homme de métier, à partir de matériel JAP 7 et JAP 9 (Jour Après Pollinisation).

La quantification des différentes bandes identifiées sur gel de migration révèle des contributions relatives de 18 %, 53 % et 29 % en moyenne, pour les transcrits de Esr1, Esr2 et Esr3, respectivement. Le promoteur de Esr2 permet donc une expression quantitative la plus forte du gène qu'il contrôle.

### EXEMPLE 2 :

### Isolement et clonage des séquences promotrices

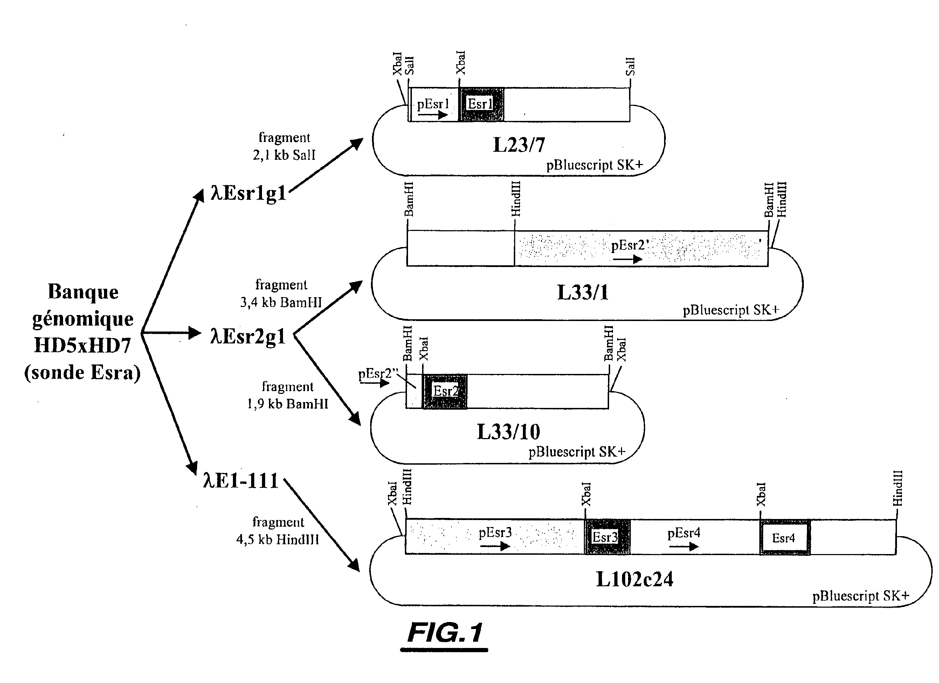

Comme illustré dans la figure 1, des fragments contenant les phases de lecture ouvertes d'Esr1, Esr2 et Esr3 (Opsahl et al, 1997) ainsi que des séquences en amont et en aval ont été sous-clonés dans le plasmide pBluescript SK+ (Stratagene) selon les méthodes décrites dans Sambrook et al (1989). Ont résulté le plasmide L23/7 contenant un fragment SalI de 2,1 kb de λEsr1g1, le plasmide L33/1 contenant un fragment BamHI de 3,4 kb de λEsr2g1, le plasmide L33/10 contenant un fragment BamHI de 1,9 kb de λEsr2g1 et le plasmide L102c24 contenant un fragment Hindlll de 4,5 kb de λE1-111. Des sites Xbal situés juste en amont de la phase de lecture ouverte (TCTAGATTCCATG) ont permis de différencier les promoteurs putatifs des phases de lecture ouvertes respectives. En particulier, le fragment SalI/XbaI de 0,53 kb de L23/7 a été désigné comme promoteur putatif d'Esr1, le fragment HindIII/BamHI de 2,35 kb.de L33/1 (partie amont du promoteur) et le fragment BamHI/XbaI de 0,14 kb de L33/10 (partie aval du promoteur), celui du promoteur putatif d'Esr2, le fragment HindIII/XbaI de 1,71 kb, celui du promoteur putatif d'Esr3 et le fragment Xbal/Xbal de 1,62 kb comprenant le promoteur putatif d'Esr4. Un promoteur Esr2 fonctionnel de 2,49 kb a été reconstitué à partir du fragment HindIII/BamHI de 2,35 kb de L33/1 et du fragment BamHI/XbaI de 0,14 kb de L33/10, dans un plasmide de base de type pBSSK+ (Stratagène). L'orientation des flèches sur la figure 1 représente l'orientation 5'-3'.

### EXEMPLE 3 :

### Structure des séquences en amont des gènes Esr

Des comparaisons entre les séquences des régions 5' ont montré deux types d'homologies : une séquence hautement conservée qui correspond à une séquence proximale de 265 paires de bases et des séquences de rétrotransposons dans la partie distale. Comme les séquences de rétrotransposons sont dans des orientations et positions différentes dans les trois promoteurs, elles ne semblent pas jouer un rôle dans l'expression des gènes Esr. Par conséquent, les 265 paires de bases contiendraient tout l'information *cis* nécessaire à une expression de gènes spécifique de la région entourant l'embryon.

La séquence consensus (SEQ ID n° 7) a été obtenue après alignement des trois séquences nucléotidiques promotrices et utilisation du logiciel Sequencher 3.1 de Genes Codes Corporation (Ann. Arbor, MI 48106).

Les bases dégénérées sont décrites dans Nomenclature Committee of the International Union of Biochemistry (1985) : Nomenclature for incompletely specified bases in nucleic acid sequences. European Journal of Biochemistry 150:1-5.
En particulier,
B=C, G ou T mais pas A
D=A, G ou T mais pas C
H=A, C ou T mais pas G
K=G ou T
M=A ou C
N=G, A, T ou C
R=G ou A
S=C ou G
V=A, C, ou G mais pas T
W=A ou T
X=G, A, T ou C
et Y=C ou T.

On observe également une homologie entre les régions proximales qui s'étend sur environ 500 paires de bases entre le promoteur de *Esr2* et celui de *Esr3,* comme défini par les séquences SEQ ID n° 5 et n° 6.

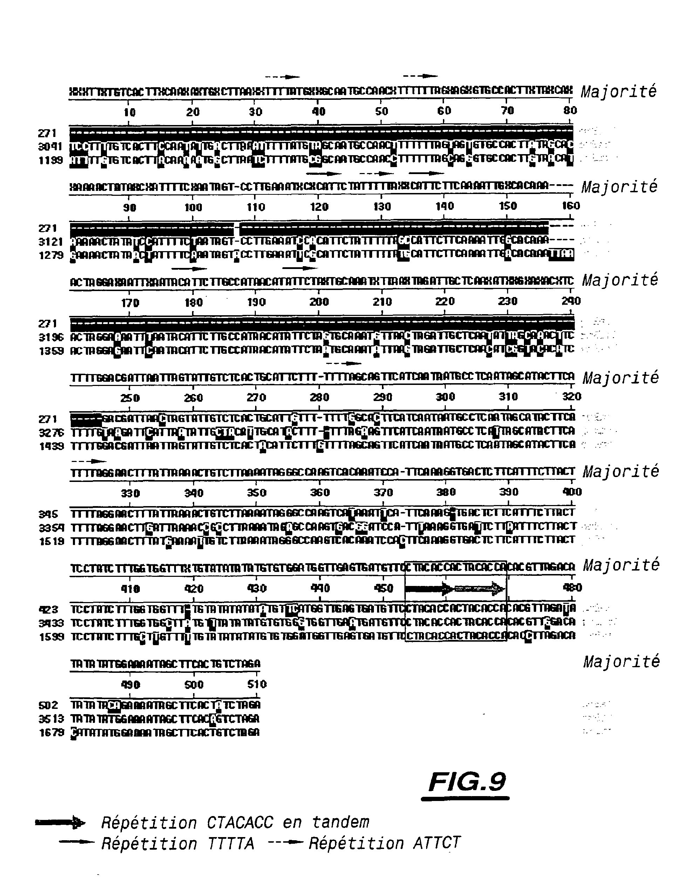

La présence d'éléments agissant en cis est recherchée parmi les séquences conservées, la plus remarquable étant CTACACCA, en tandem juste 50 bases en amont de la phase ouverte de lecture (figure 9). Cette séquence est un bon candidat pour être un élément responsable d'une expression génique tissu-spécifique. La première de ces répétitions est d'ailleurs placée dans la boucle de la répétition inversée la plus importante trouvée dans les trois promoteurs. Les séquences répétées plus de deux fois ne sont conservées qu'entre les promoteurs *pEsr2* et *pEsr3,* dans la région manquante de *Esr1 :* par exemple les séquences ATTCT et TTTTA, chacune étant répétée quatre fois, potentiels enhancers de transcription au vu de l'expression plus faible de Esr1 (figure 9).

Pour démontrer la fonctionnalité des éléments *cis*, des construits comprenant des séquences nucléotidiques promotrices délétées, fusionnées à *GUS,* ont été préparées.

A titre d'exemple, deux techniques ont été utilisées pour créer des délétions du promoteur Esr2 :
- par digestion extensive de 5' vers 3' sur le fragment Hindlll-Xbal à l'aide du kit Erase-a-base™ de Promega (constructions L140).
- par amplification PCR de fragments du promoteur (constructions L194).

Les plasmides L140 et L194 contiennent des promoteurs Esr2 délétés fusionnés à un gène rapporteur Gus et un terminateur, selon les techniques décrites à l'exemple suivant.

Pour l'étape de transformation, les fragments contenant les construits 'promoteurs Esr2 délétés - Gus -ter' ont été transférés dans un autre plasmide contenant le construit 'promoteur ubiquitin-luciferase-ter', ce dernier servant de standard interne pour quantifier l'activité Gus et corriger l'effet position de l'insertion du transgène dans le génome sur l'expression, variable d'une plante transformée à l'autre.

Sont répertoriés dans le tableau 1 suivant les fragments du promoteur Esr2 issus de ces délétions.

**Tableau 1:**

| Technique de délétion choisie | Fragment restant du promoteur Esr2* |
|---|---|
| Digestion kit (L140) | 985-2493 |
| | 1254-2493 |
| | 1865-2493 |
| | 1874-2493 |
| | 1880-2493 |
| | 2077-2493 |
| Amplification PCR (L194) | 2163-2493 |
| | 2275-2493 |
| | 2373-2493 |

| | |
|---|---|
| * La numérotation du promoteur Esr2 est basée sur la séquence pEsr2 présentée en annexe (SEQ ID n° 2), qui va de Hindlll (AAGCTT ou A = position 1) à Xbal (TCTAGA ou A = position 2493) | |

Pour démontrer la fonctionnalité des séquences nucléotidiques promotrices décrites ci-dessus, les inventeurs les ont clonées en amont du gène rapporteur GUS et ont utilisé les construits obtenus pour la transformation de plantes.

De manière préférée, les promoteurs délétés *Esr2* ont été obtenus selon les protocoles suivants :
Premièrement, des délétions de 5' vers 3' ont été effectuées à l'aide de l'exonucléase III. Le plasmide L124/19 contenant le promoteur du gène *Esr2* couplé au gène de la ß-glucuronidase décrit à l'exemple 4.1 a été digéré par HindIII pour générer un site d'initiation des délétions et par PstI pour créer un site de protection contre l'action de l'exonucléase III. Les délétions ont été effectuées avec le kit Erase-a-base™ (Promega).
Deuxièmement, des fragments de promoteur ont été amplifiés à l'aide de l'amorce ESRX (5'GGGGTCTAGACTGTGAAGCTATTTTCCA3' (SEQ ID n° 8)) contenant le site de restriction Xbal (souligné) et ESRH1 (5'GGGGAAGCTTTACATTCTTGCCATAACATA3' (SEQ ID n° 9)), ESRH2 (5'GGGGAAGCTTTTCATCAATAATGCCTCATT3' (SEQ ID n° 10)) ou ESRH3 (5'GGGGAAGCTTTAATTTCTTACTTCCTATCT3'-(SEQ ID n° 11)) contenant le site de restriction HindIII (souligné). Les produits d'amplification digérés par Xbal et HindIII ont remplacé le promoteur *Esr2* entier en amont du gène de la ß-glucuronidase dans le plasmide L124/19.
Les promoteurs délétés associés au gène de la ß-glucuronidase ont ensuite été clonés dans un plasmide contenant le gène de la luciférase sous le contrôle du promoteur de l'actine du riz. Ce dernier a été obtenu par clonage du fragment XhoI/NcoI du plasmide pAct1-F4 (Mc Elroy D. et al., Mol Gen Genet., 231 :150-160, 1991) correspondant au promoteur et premier intron de l'actine du riz, dans un plasmide de type pGP214 contenant le gène de la luciférase et le terminateur de la nopaline synthase (Twell D. et al., Development 109, 705-713, 1990), digéré par SalI/NcoI. Un adaptateur contenant les sites de restriction SalI et Notl, formés des nucléotides 5'GGCCAGTCGACAAAGCGGCCGCATGCA3' (SEQ ID n° 12) et 5'TCAGCTGTTTCGCCGGCGT3' (SEQ ID n° 13) a été introduit dans le plasmide obtenu, digéré par Notl et PstI (plasmide L210). Les fragments SalI/NotI contenant les promoteurs délétés associés au gène de la ß-glucuronidase ont été clonés dans le plasmide L210 digéré par SalI et Notl.

### EXEMPLE 4 :

### Préparation des construits chimériques

Toutes les constructions peuvent été réalisées notamment selon les méthodes décrites dans Sambrook et al. (1989). Les adaptateurs, pouvant être utilisés à titre d'exemple pour cloner ces fragments en amont des différents gènes effecteurs, sont décrits dans les cartes de restriction des plasmides correspondantes.

### 4-1 Construits chimériques GUS

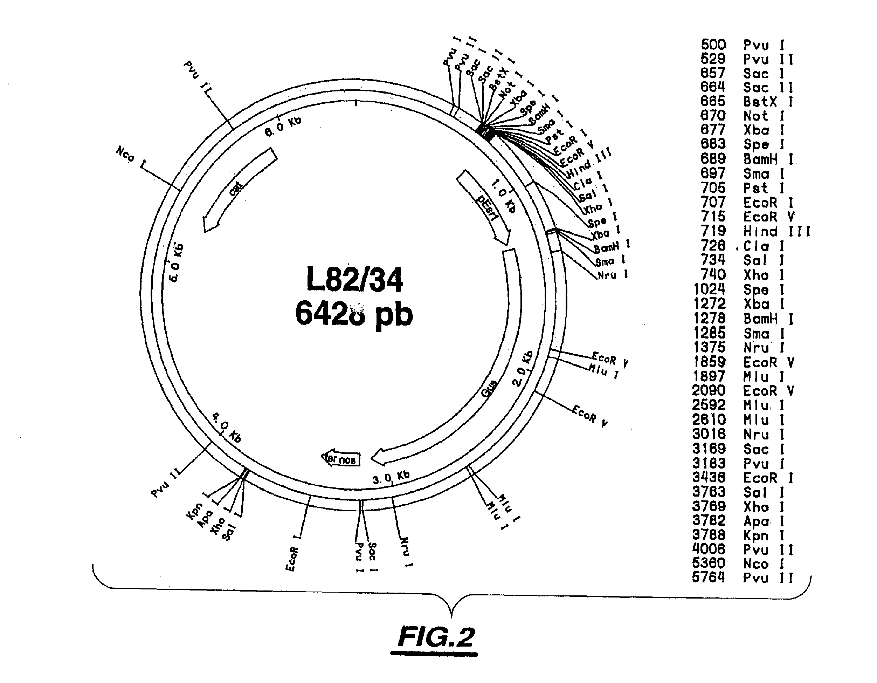

Le plasmide L23/7 (Esr1) a été délété d'un fragment SacI contenant des sites de restriction non souhaitables. Puis un fragment Xbal/EcoRI de 2164 paires de bases du plasmide pBI101 (Jefferson et al., 1987) contenant un gène *Gus* (codant pour la β-glucuronidase mais dépourvu de promoteur) et une séquence terminatrice *nos,* a été introduit. Le nouveau plasmide ainsi formé a alors été digéré par Xhol et le produit de digestion contenant la région promotrice associée au gène *Gus* et positionnée en amont de ce dernier, a été sous cloné dans le vecteur pBCKS+ (Stratagene) de manière à ce que le promoteur soit à proximité de la zone d'hybridation de l'amorce T7, permettant ainsi l'obtention du plasmide L82/34 (figure 2, tableau 2).

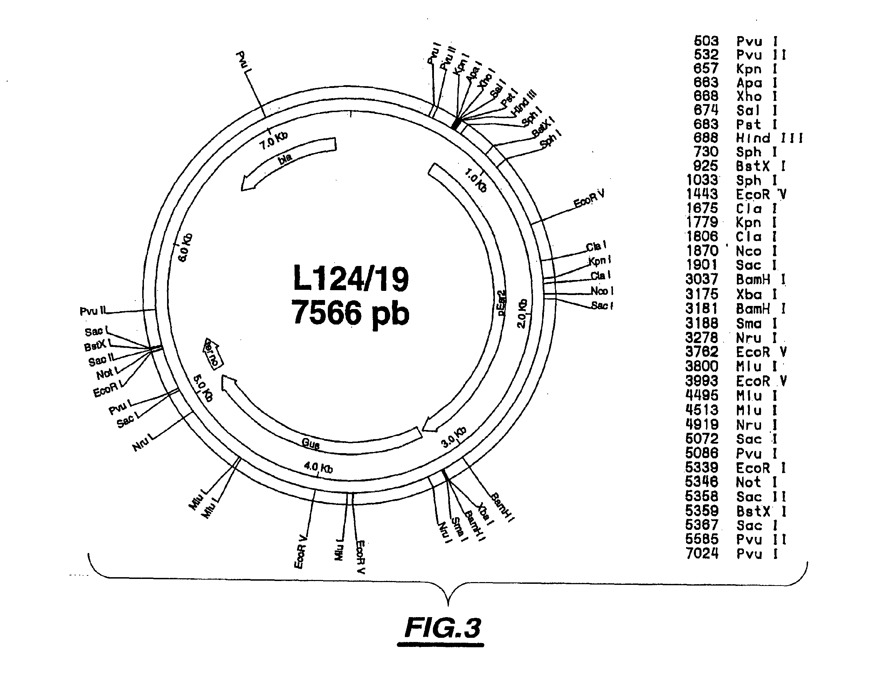

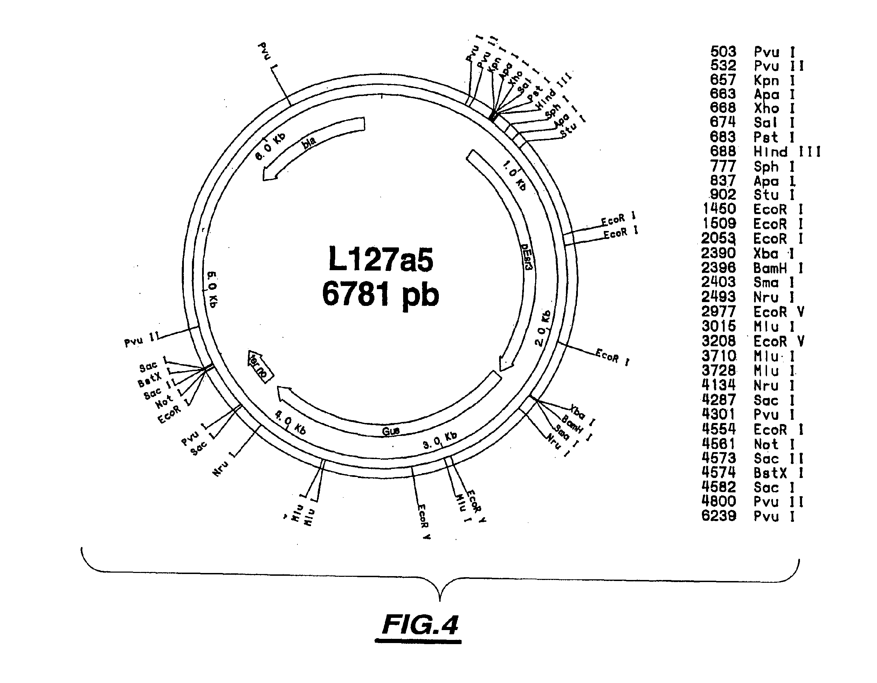

Selon un protocole semblable et avec les enzymes de restriction indiquées dans les figures correspondantes, ont pu être obtenus les plasmides L124/19 (pEsr2-GUS, figure 3, tableau 3) et L127a5 (pEsr3-GUS, figure 4, tableau 4).

Il est également possible d'utiliser d'autres gènes rapporteurs en remplacement de GUS, par exemple la GFP (Green Fluorescent Protein, Siemering KR et al., 1996), pour confirmer les résultats obtenus avec GUS. Selon un protocole semblable à celui décrit précédemment, le fragment Hindlll-Xbal du promoteur pEsr2 a été fusionné à la séquence codant pour la GFP.

**Tableau 2 : Caractéristiques du plasmide L82/34**

| Fragment | Position | Référence |
|---|---|---|
| pEsr1 | 741-1272 | |
| Gus | 1302-3107 | |
| ter nos | 3181-3434 | |
| cat | 5878-5223 | |
| pBCKS+ | 1-740 | Stratagene |
| L23/7 (pEsr1) | 741-1272 | Opsahl-Ferstad et al, 1997* et cet exemple |
| pBI101 | 1273-3436 | Jefferson et al, 1987 |
| L23/7 (en amont) | 3437-3763 | Opsahl-Ferstad et al, 1997 et cet exemple |
| pBSSK+ | 3764-3769 | Stratagene |
| pBCKS+ | 3770-6428 | Stratagene |

| | | |
|---|---|---|
| * l'insert correspond au fragment Esr1g1 dessiné dans la figure 4 d'Opsahl-Ferstad et al, 1997 | | |

**Tableau 3 : Caractéristiques du plasmide L124/19**

| Fragment | Position | Référence |
|---|---|---|
| pEsr2 | 689-3175 | |
| Gus | 3205-5010 | |
| ter nos | 5084-5337 | |
| bla | 7441-6584 | |
| pBSSK+ | 1-674 | Stratagene |
| Linker JFB 34 | 675-688 | cet exemple ¹⁾ |
| L33/1 (pEsr2') | 689-3037 | cet exemple ** |
| L33/10 (pEsr2") | 3038-3175 | Opsahl-Ferstad et al, 1997 et cet exemple** |
| pBI101 | 3176-5339 | Jefferson et al, 1987 |
| linker JFB 5 6 | 5340-5346 | cet exemple ²⁾ |
| pBSSK+ | 5347-7566 | Stratagene |

| | | |
|---|---|---|
| ** les inserts sont présentés en partie comme fragment Esr2g1 dans la figure 4 de Opsahl-Ferstad et al, 1997 ¹⁾ adaptateur JFB34 : 5' TCGACTGCAGCCCA 3' (SEQ ID n° 14) 3' GACGTCGGGTTCGA 5' (SEQ ID n° 15) ²⁾ adaptateur JFB56 : 5' CTAGACCCGAATTCGC 3' (SEQ ID n° 16) 3' TGGGCTTAAGCGCCGG 5' (SEQ ID n° 17) | | |

**Tableau 4 : Caractéristiques du plasmide L127a5**

| Fragment | Position | Référence |
|---|---|---|
| pEsr3 | 689-2390 | |
| Gus | 2420-4225 | |
| ter nos | 4229-4552 | |
| bla | 6656-5799 | |
| Pbssk+ | 1-674- | Stratagene |
| Linker JFB 34 | 675-688 | cet exemple |
| L102c24 (pEsr3) | 689-2390 | cet exemple |
| pBI101 | 2391-4554 | Jefferson et al, 1987 |
| linker JFB56 | 4555-4561 | cet exemple |
| pBSSK+ | 4562-6781 | Stratagene |

### 4-2 Construits chimériques Ipt

Le gène Ipt code pour l'isopentényl-transférase, qui est une enzyme impliquée dans la synthèse de la cytokinine, phytohormone jouant un rôle dans la croissance cellulaire végétale. La séquence du gène a été déterminée par Heidekamp F. et al (1983). Des travaux antérieurs ont montré par ailleurs que cette séquence, sous le contrôle d'un promoteur spécifique de l'ovule permettait d'augmenter le contenu en matière sèche dans le fruit, chez la tomate Martineau B. et al. (1995).

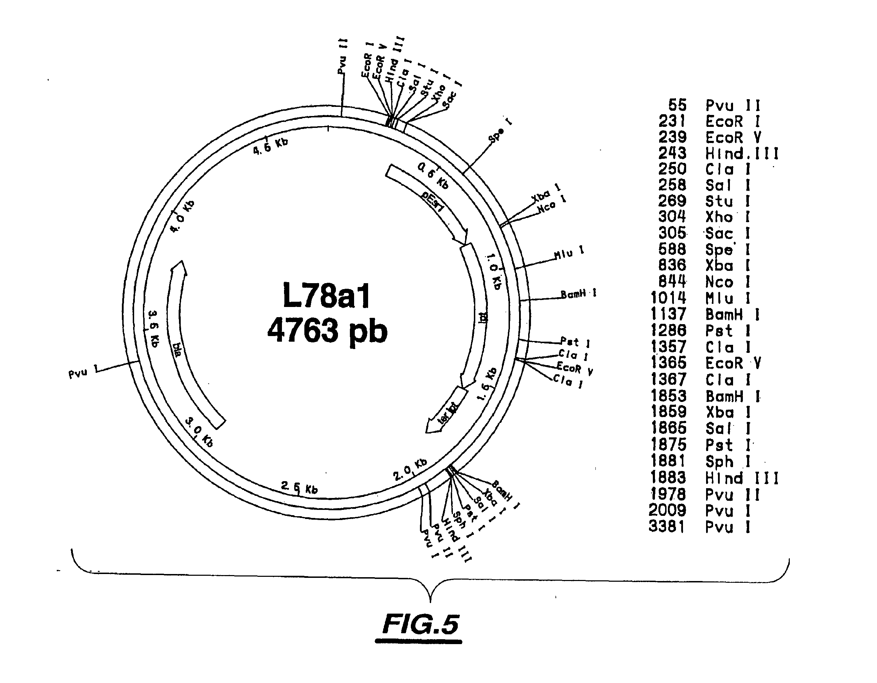

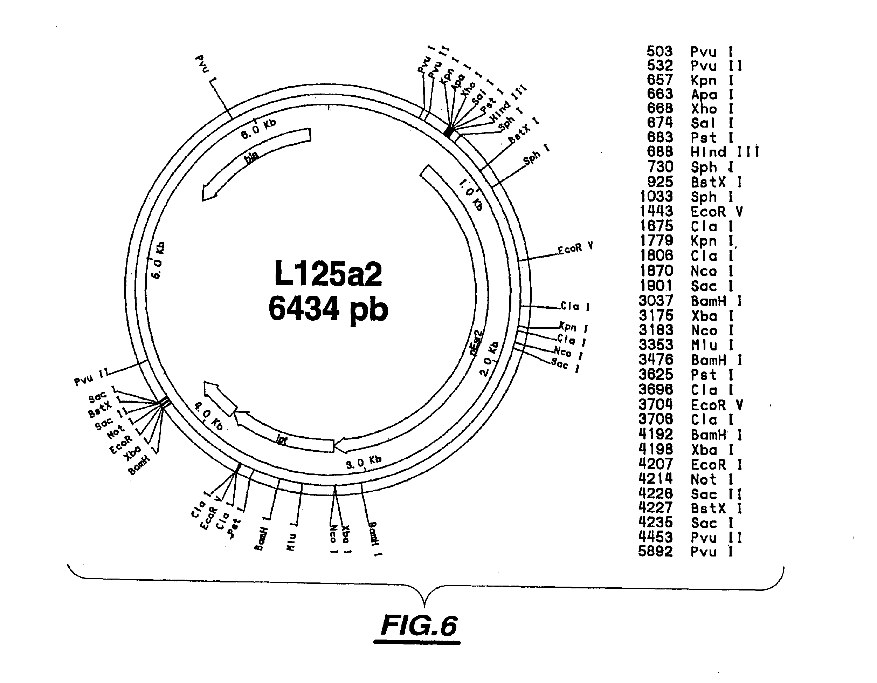

Selon les méthodes de clonage décrites ci-dessus et avec les fragments d'acides nucléiques et enzymes de restriction indiquées dans les figures correspondantes, ont pu être préparés des construits pEsr1-Ipt (figure 5, tableau 5) et pEsr2-Ipt (figure 6, tableau 6). Il est également possible d'obtenir un construit pEsr3-Ipt, selon le même protocole. Pour la préparation de ces construits, des sites Ncol (CCATGG) chevauchant le codon ATG du début de la phase de lecture ouverte ont été utilisés au lieu des sites Xbal.

**Tableau 5 : Caractéristiques du plasmide L78a1**

| Fragment | Position | Référence |
|---|---|---|
| pEsr1 | 310-844 | |
| ipt | 846-1565 | |
| ter ipt | 1566-1850 | |
| bla | 2963-3823 | |
| pUC118 | 1-231- | Boehringer |
| L23/7 (pEsr1) | 232-844 | Opsahl-Ferstad et al, 1997 et cet exemple |
| mutation ponctuelle | 845 | Zhang et al, 1996 |
| pRZ1 | 846-1883 | Zhang et al, 1995 |
| pUC118 | 1884-4763 | Boehringer |

**Tableau 6 : Caractéristiques du plasmide L125a2**

| Fragment | Position | Référence |
|---|---|---|
| pEsr2 | 689-3183 | |
| ipt | 3185-3904 | |
| ter ipt | 3905-4189 | |
| bla | 6309-5449 | |
| pBSSK+ | 1-674 | Stratagene |
| Linker JFB 34 | 675-688 | cet exemple |
| L33/1 (pEsr2') | 689-3037 | cet exemple |
| L33/10 (pEsr2") | 3038-3183 | Opsahl-Ferstad et al, 1997 et cet exemple |
| mutation ponctuelle | 3184 | Zhang et al, 1996 |
| pRZ1 | 3185-4198 | Zhang et al, 1995 |
| adaptateur JFB56 | 4199-4214 | cet exemple |
| pBSSK+ | 4215-6434 | Stratagene |

### 4-3 Construits chimériques barnase

Le gène barnase code pour une RNase. Ce gène a été isolé à partir de Bacillus amyloliquefaciens (Hartley, 1988). Son utilisation pour créer des plantes mâles stériles a été décrite dans la demande EP 344 029 et publiée par Mariani et al (1990).

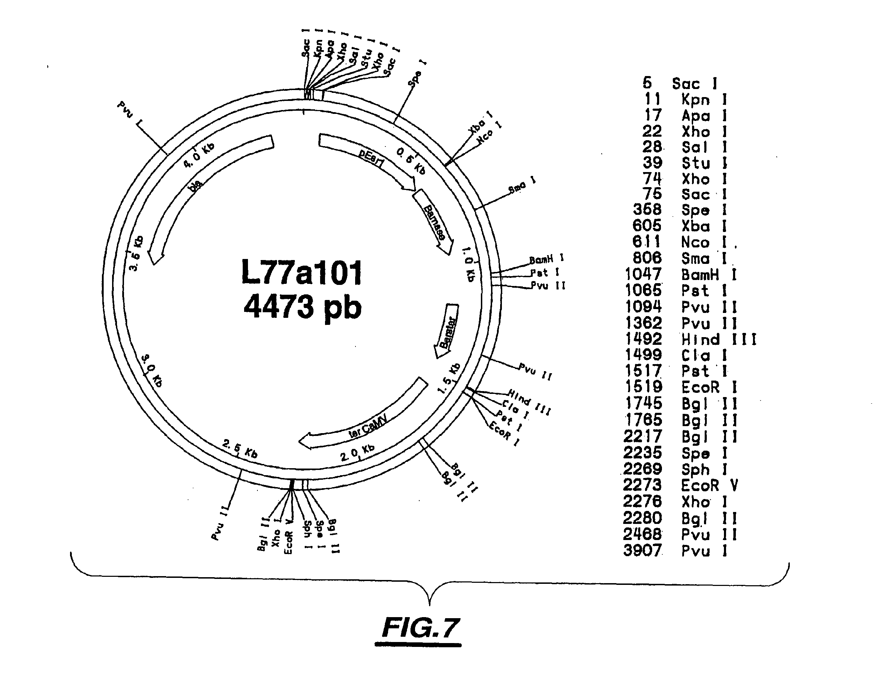

Dans le cadre de l'invention, les plasmides L77a101 (pEsr1∼barnase) et L126a3 (pEsr2~barnase) décrits aux figures 7 (tableau 7) et 8 (tableau 8) ont été obtenus à partir du plasmide 'promoteur A6~barnase' décrit dans WO 92/11379, par le remplacement du pA6 par les promoteurs pEsr1 et pEsr2 respectivement, selon les techniques connues de l'homme de métier.

On peut également obtenir un construit pEsr3-Barnase, selon un protocole semblable.

**Tableau 7 : Caractéristiques du plasmide L77a101**

| Fragment | Position | Référence |
|---|---|---|
| pEsr1 | 80-605 | |
| Barnase | 613-945 | |
| ter CaMV | 1571-2257 | |
| bla | 4324-3467 | |
| pA3 | I-28 | Scott et al, 1992 |
| L23/7 (pEsr1) | 29-605 | Opsahl-Ferstad et al, 1997 et cet exemple |
| pA3 | 606-4473 | Scott et al, 1992 |

**Tableau 8 : Caractéristiques du plasmide L126a3**

| Fragment | Position | Référence |
|---|---|---|
| pEsr2 | 43-2529 | |
| Barnase | 2537-2869 | |
| ter CaMV | 3495-4181 | |
| bla | 6248-5391 | |
| pA3 | I-28 | Scott et al, 1992 |
| linker JFB34 | 29-42 | cet exemple |
| L33/1 (pEsr2') | 43-2391 | cet exemple |
| L33/10 (pEsr2") | 2392-2529 | Opsahl-Ferstad et al, 1997 et cet exemple |
| pA3 | 2530-6397 | Scott et al, 1992 |

### 4-4 Construit chimérique antiEsr2

Le promoteur Esr2 fonctionnel reconstitué (2,49kb) décrit à l'exemple 2 et choisi préférentiellement au vu des résultats d'expression quantitative décrits à l'exemple 1, a été fusionné à la séquence Esr2g2 (Opsahl et al., 1997) prise en orientation antisens, elle-même fusionnée au terminateur Nos.

De manière préférée, le construit chimérique contenant le gène *Esr2* en antisens sous contrôle de son propre promoteur a été obtenu selon le protocole suivant :
Dans un plasmide dérivé du pJIT30 contenant le promoteur 35S, un site multiple de clonage et la séquence terminateur du virus de la mosaïque du chou (Guerineau F. et al., Plant Mol Biol, 15: 127-136, 1990), un adaptateur contenant un site SpeI et formé des oligonucléotides (5'GATCCACTAGTCCCG (SEQ ID n° 18)) et (5'AATTCGGGACTAGTG (SEQ ID n° 19)) a été inséré entre des sites BamHI et EcoRI. Le fragment EcoRI/SpeI du plasmide L42 a14 (Opsahl-Ferstad et coll., 1997) a été inséré dans le plasmide décrit précédemment. La construction ainsi obtenue contenait le gène *Esr2* en orientation antisens sous le contrôle du promoteur 35S (plasmide L79 b5).

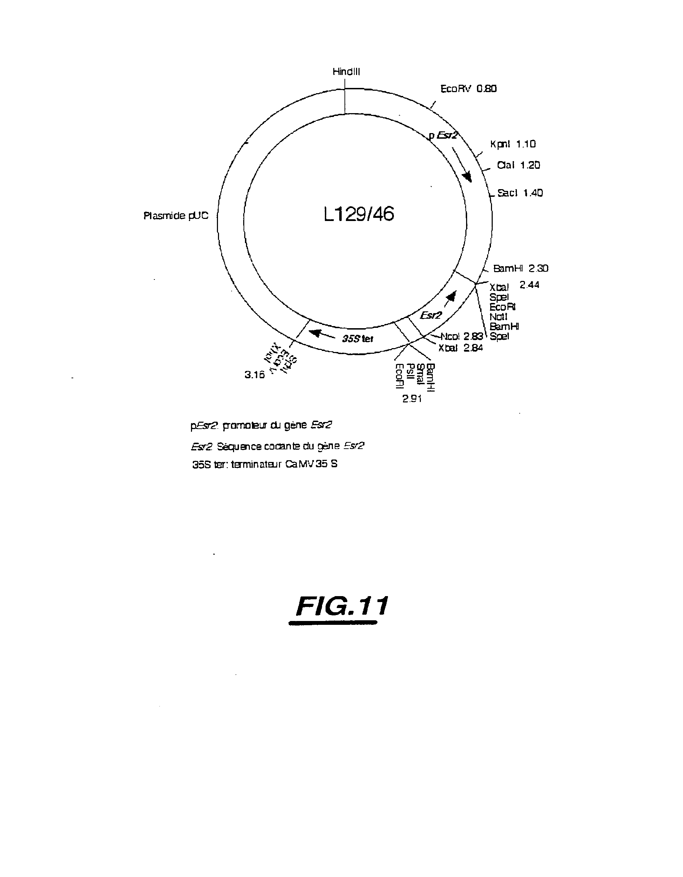

Le promoteur 35S a été supprimé dans le plasmide L79 b5 par restriction par Sacl et HindIII, et remplacé par un adaptateur contenant les sites de restriction Hindlll et Notl et formé des oligonucléotides (5'AAGCTTTTTGCGGCCGC (SEQ ID n° 20)) et (5'TCGAGCGGCCGCAAAAAGCTTAGCT (SEQ ID n° 21)). Le promoteur *Esr2* sous forme d'un fragment HindIII/NotI de 2,44 kb a été introduit dans cet adaptateur. La construction ainsi obtenue contient le gène *Esr2* en orientation antisens sous le contrôle de son propre promoteur (plasmide L129/46 (cf figure 11)).

Selon un protocole semblable, on peut obtenir les construits comprenant le promoteur Esr2 fusionné aux séquences antisens Esr1 et Esr3 respectivement. Il est également possible d'obtenir le même type de construits chimériques avec les autres promoteurs Esr selon l'invention.

Des construits comprenant le promoteur constitutif 35S fusionné aux séquences antisens Esr décrites ci-dessus ont également été obtenus.

### EXEMPLE 5 :

### Obtention de plantes transgéniques (nécessité de la transformation stable du maïs)

Des expériences d'expression transitoire utilisant la transformation par bombardement de cellules végétales, avec des construits chimériques pEsr~GUS et promoteur constitutif~Gus respectivement, n'ont pas donné de résultats mettant en évidence la spécificité d'expression des promoteurs testés : aucune activité GUS n'a été visualisée dans la zone définie par les cellules Esr. La petite taille de cette zone et d'autres particularités propres pourraient expliquer le fait que la technique est inadaptée en conditions standards pour une expression transitoire. A titre d'exemple, les promoteurs constitutifs testés en contrôle sont les promoteurs actine de riz (McElroy et al., 1992), ubiquitine de maïs (Christensen et al., 1996), Adh de maïs (Dennis et al., 1984) et 35S (Odell et al., 1985), ont donné une coloration bleue dans tout l'albumen, démontrant la fonctionnalité du système de transformation, mais pas dans la zone entourant l'embryon, ce qui confirme l'inadaptation du système à cette zone.

La transformation visant une expression stable devenait donc nécessaire pour étudier la spécificité d'expression des promoteurs selon l'invention.

### 5-1 Canon à particules

La méthode utilisée repose sur l'utilisation d'un canon à particules identique à celui décrit par J. Finer (1992). Les cellules cibles sont des cellules indifférenciées en divisions rapides ayant conservé une aptitude à la régénération de plantes entières. Ce type de cellules compose le cal embryogène (dit de type II) de maïs. Ces cals sont obtenus à partir d'embryons immatures de génotype Hill selon la méthode et sur les milieux décrits par Armstrong (Maize Handbook ; 1994 M. Freeling, V. Walbot Eds ; pp.665-671). Ces fragments des cals d'une surface de 10 à 20 mm² ont été disposés, 4h avant bombardement, à raison de 16 fragments par boite au centre d'une boîte de Petri contenant un milieu de culture identique au milieu d'initiation, additionné de 0,2 M de mannitol + 0,2 M de sorbitol. Les plasmides décrits dans les exemples précédents et portant les gènes à introduire, sont purifiés sur colonne Qiagen^{R} en suivant les instructions du fabricant. Ils sont ensuite précipités sur des particules de tungstène (M10) en suivant le protocole décrit par Klein (1987). Les particules ainsi enrobées sont projetées vers les cellules cibles à l'aide du canon et selon le protocole décrits par J. Finer (1992). Les boîtes de cals ainsi bombardées sont ensuite scellées à l'aide de Scellofrais^{R} puis cultivées à l'obscurité à 27°C. Le premier repiquage a lieu 24h après, puis tous les quinze jours pendant 3 mois sur milieu identique au milieu d'initiation additionné d'un agent sélectif. On obtient après 3 mois ou parfois plus tôt, des cals dont la croissance n'est pas inhibée par l'agent sélectif, habituellement et majoritairement composés de cellules résultant de la division d'une cellule ayant intégré dans son patrimoine génétique une ou plusieurs copies du gène de sélection. La fréquence d'obtention de tels cals est d'environ 0,8 cal par boîte bombardée.

Ces cals sont identifiés, individualisés, amplifiés puis cultivés de façon à régénérer des plantules, en modifiant l'équilibre hormonal et osmotique des cellules selon la méthode décrite par Vain et al. (1989). Ces plantes sont ensuite acclimatées en serre où elles peuvent être croisées pour l'obtention d'hybrides ou autofécondées.

### 5-2. Transformation par Agrobacterium

Une autre technique de transformation utilisable dans le cadre de l'invention utilise *Agrobacterium tumefaciens,* selon le protocole décrit par Ishida et al (1996), notamment à partir d'embryons immatures de 10 jours après la fécondation. Tous les milieux utilisés sont référencés dans la référence citée. La transformation débute avec une phase de co-culture où les embryons immatures des plantes de maïs sont mis en contact pendant au moins 5 minutes avec *Agrobacterium tumefaciens* LBA 4404 contenant les vecteurs superbinaires. Le plasmide superbinaire est le résultat d'une recombinaison homologue entre un vecteur intermédiaire porteur de l'ADN-T contenant le gène d'intérêt et/ou le marqueur de sélection dérivé des plasmides décrits dans les exemples précédents, et le vecteur pSB1 de Japan Tobacco (EP 672 752) qui contient : les gènes virB et virG du plasmide pTiBo542 présent dans la souche supervirulente A281 *d'Agrobacterium tumefaciens* (ATCC 37349) et une région homologue retrouvée dans le vecteur intermédiaire permettant cette recombinaison homologue. Les embryons sont ensuite placés sur milieu LSAs pendant 3 jours à l'obscurité et à 25°C. Une première sélection est effectuée sur les cals transformés : les cals embryogènes sont transférés sur milieu LSD5 contenant de la phosphinotricine à 5 mg/l et de la céfotaxime à 250 mg/l (élimination ou limitation de la contamination par *Agrobacterium tumefaciens).* Cette étape est menée 2 semaines à l'obscurité et à 25°C. La deuxième étape de sélection est réalisée par transfert des embryons qui se sont développés sur milieu LSD5, sur milieu LSD10 (phosphinotricine à 10 mg/l) en présence de céfotaxime, pendant 3 semaines dans les mêmes conditions que précédemment. La troisième étape de sélection consiste à exciser les cals de type I (fragments de 1 à 2 mm) et à les transférer 3 semaines à l'obscurité et à 25°C sur milieu LSD 10 en présence de céfotaxime.

La régénération des plantules est effectuée en excisant les cals de type I qui ont proliféré et en les transférant sur milieu LSZ en présence de phosphinotricine à 5 mg/l et de céfotaxime pendant 2 semaines à 22°C et sous lumière continue.

Les plantules ayant régénéré sont transférées sur milieu RM + G2 contenant 100mg/l d'Augmentin pendant 2 semaines à 22°C et sous illumination continue pour l'étape de développement. Les plantes obtenues sont alors transférées au phytotron en vue de leur acclimatation.

### 5-3 Mode préféré pour les construits barnase : re-transformation de cals act-barstar

Les construits chimériques barnase décrits à l'exemple 3 peuvent être utilisés pour des transformations classiques selon l'une ou l'autre des techniques décrites précédemment.

Selon un mode préféré, adapté au caractère toxique de la barnase, on utilise pour la transformation des cals pré-transformés contenant le gène barstar, qui code pour un inhibiteur spécifique de la Barnase (Hartley, 1988). Ce gène sert de 'protection' pendant le processus de régénération de ces cals, qui se fait essentiellement à partir de l'embryogénèse chez le maïs.
- étape a : obtention d'une lignée exprimant le barstar et un plasmide contenant le gène de résistance à l'hygromycine : Une première étape de transformation est réalisée selon l'un des protocoles décrits, avec le plasmide pWP280 contenant la cassette pActin-intron-Barstar-Nos poly A.

Cette cassette a été obtenue selon les étapes suivantes : le fragment barnase a été amplifié par PCR à partir du plasmide pTG2 (Horovitz et al, 1990) puis sous-cloné en tant que fragment Xba1/HindIII dans le plasmide pBluescript KS+ (Stratagene) donnant le plasmide pWP118.

Le gène barstar a ensuite été transféré en tant que fragment Xba1/HincII dans un site Xba1/Sma1 du plasmide pW90, dérivé du plasmide pJIT30 décrit par Guerineau et al (1990) (promoteur 35SCaMV remplacé par le promoteur double 35S et la région *polylinker* entre les sites Xba1 et EcoR1 remplacée par les sites Spe1, BamH1, Sma1 et Pst1).

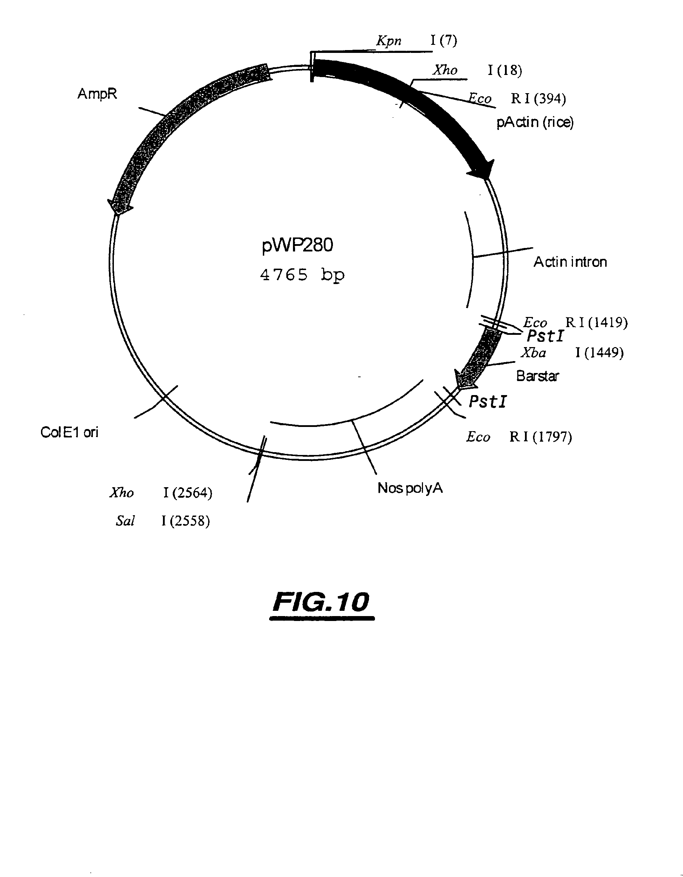

La région polyA CaMV du plasmide obtenu est remplacée par la région nos polyA de pED23 (Dale et al, 1991) formant le plasmide pWP266. La région promotrice double 35S CaMV est enfin remplacée par le promoteur actine du riz et l'intron issu de pCOR113 (Mc Elroy et al, 1991) formant le plasmide pWP280 (figure 10).

Les plantes "promoteur actine-barstar" ainsi produites sont analysées par Northern Blot pour identifier les plantes exprimant correctement l'ARNm codant pour Barstar. Les plantes ainsi produites fournissent des embryons exprimant le gène Barstar, qui serviront à la production de cals de type II selon les techniques connues : mise en culture des embryons sur milieu d'induction de la callogénèse et repiquage sur milieu sélectif contenant de l'hygromycine.

### - étape b : transformation de ces cals avec le construit chimérique barnase :

Les cals act-barstar obtenus à l'étape précédente sont ensuite bombardés selon la technique décrite au point 5-1 par les construits 'promoteur Esr~barnase' précédemment décrits avec un plasmide conférant la résistance au Basta (pDM302, Mc Elroy et al, 1991). On sépare ensuite les deux gènes dans la descendance par ségrégation, pour voir l'effet du seul construit promoteur Esr~barnase.

De meilleurs résultats, notamment en ce qui concerne l'efficacité de transformation et le nombre de plantes régénérées, ont été obtenus selon ce mode préféré, en comparaison à la technique classique qui vise à transformer directement les cals par les construits 'Esr∼barnase'.

### EXEMPLE 6 :

### Mise en évidence de la fonctionnalité des séquences promotrices (cas des construits GUS)

Afin de détecter l'activité β-glucuronidase, les grains de maïs issus de plantes transformées par le canon à particules sont récoltés à des stades précis du développement et coupés le long de l'axe longitudinal. Ils sont incubés en présence de 5-bromo-4-chloro-3-indolyl-β-D-glucuronic acid (X-GlcA, Duchefa), à 37°C pendant 24 heures (Jefferson et al., 1987).

Dans le cas de construit Esr2-Gus notamment, la coloration bleue est délimitée au contour de l'embryon au 4^{ème} et 5^{ème} jour après pollinisation, puis seulement au niveau suspenseur les 6^{ème} et 7^{ème} jours, et enfin à la base du suspenseur aux jours 9,12, 13 et 15.

Ces résultats d'expression dans les plantes transgéniques démontrent que les fragments 5' décrits dans la présente invention correspondent à des promoteurs fonctionnels et qu'ils sont suffisants pour une expression spatio-temporelle correcte, en accord avec les résultats antérieurs de Opsahl et al (1997).

L'utilisation de ces séquences nucléotidiques promotrices dans des constructions moléculaires destinées à améliorer la qualité agronomique, alimentaire ou industrielle d'une plante, est particulièrement intéressante pour modifier la taille de l'embryon ou de l'albumen et/ou son développement.

### BIBLIOGRAPHIE

- Armstrong (1994). Maize Handbook ; Freeling M., Walbot V. Eds ; 665-671.
- Barloy, D., et coll., (1989). Maydica, 34, 303-308.
- Becker, H. A., et al., (1999). Domains of gene expression in developing endosperm. 361-375
- Breton, C., et al., (1995). Plant. Mol. Biol. 27, 105-113.
- Christensen et al., (1996), Transgenic Res., 5 : 213.
- Clark, J. K., et Sheridan, W. F. (1986). J. Heredity. 77, 83-92.
- Dale et al, (1990) Gene 91:79-85
- Davis, R. W., et coll., (1990). Can. J. Bot. 68, 471-479.
- Dennis, (1884), Nucl. Ac. Res., 12 : 3983-4000.
- Devic et al., (1997), Plant Physiol. Biochem., 35 : 35(4): 331-339
- Finer J., (1992). Plant Cell Report, 11 : 323-328.
- Gerdes, J. T., et Tracy, W. F.(1993). Crop Sci. 33, 334-337.
- Guerche et al, (1987). Mol. Gen. Genet. 206:382
- Guerineau et al, (1990), Plant. Mol. Biol. 15:127-136
- Hartley et al., (1988), J. Mol. Biol., 202, 913-915.
- Heidekamp F. et al, (1983), Nucl Acids Res 11, 6211-6223.
- Horovitz et al, (1990), J. Mol. Biol. 216:1031-1044
- Hu et al., (1995), Molecular and General Genetics, 248:471-480
- Hueros, G., et al., (1995). Plant Cell, 7, 747-757.
- Ishida et al., (1996), Nature Biotechnology, 14 :745-750
- Jefferson et al., (1987), Plant Molecular Biology Reporter, 5(4): 387-405
- Klein, (1987). Nature, 327 : 70-73.
- Kowles, R. V., et Phillips, R. L., (1988). Int. Rev. Cytol. 112, 97-136.
- Kyle, D. J., et Styles, E. D., (1977). Planta. 137, 185-193
- Liang, P., et Pardee, A. B., (1992). Science, 257, 967-971.
- Lopes, M. A., et Larkins, B. A., (1993). Plant Cell, 5, 1383-1399.
- Mariani et al. (1990), Nature 347, 737-741.
- Mc Elroy et al, (1991) Mol. Gen. Genet. 231:150-160
- McElroy et al., Plant Cell, 2 : 163-171, 1990.
- Odell et al., (1985), Nature 313 : 810-812.
- Opsahl-Ferstad, H. D., et al., (1997). The Plant J., 12, 235-246.
- Poole et al., (1985), Cell, 40 :37-43
- Sambrook et al., (1989), Molecular Cloning - A laboratory manual ; Cold Spring Harbor Laboratory Press
- Schel, J. H. N., et coll., (1984). Can. J. Bot. 62, 2842-2856.
- Scott et al, (1992), demande de brevet WO 92/11 379
- Siemering KR. et al., (1996), Current Biology 6, 1653-1663
- Twell D. et al., Development 109, 705-713, 1990
- Vain et al., (1989). Plant Cell Tissue and Organ Culture, 18 : 143-151.
- Xu, J., et al., (1995). Plant Physiol. 108, 1293-1294.
- Zhang et al, (1995). The effect of auxin on cytokinin levels and metabolism in transgenic tobacco tissue an ipt gene. Planta 196:84-94
- Zhang et al, (1996). Expression of the isopentenyl transferase gene is reguraled by auxin in transgenic tobacco tissues, Transgenic Res. 5:57-65.

## Revendications

1. Séquence nucléotidique promotrice isolée permettant une expression des séquences codantes auxquelles elle est liée, ladite expression étant i) spécifique de la région de l'albumen entourant l'embryon dans les graines des Angiospermes et ii) intervenant en particulier dans les stades précoces du développement de l'albumen, consistant en SEQ ID N° 7 ou une séquence identique à au moins 80 % de SEQ ID N°7.

2. Séquence nucléotidique promotrice selon la revendication 1, isolée à partir de céréales, notamment de maïs.

3. Séquence nucléotidique promotrice selon l'une quelconque des revendications 1 à 2, comprenant en outre un élément régulateur en cis défini par le motif CTACACCA, de préférence répété en tandem.

4. Cassette d'expression comprenant une séquence nucléotidique promotrice selon l'une des revendications 1 à 3, liée de manière opérante à au moins un gène d'intérêt.

5. Cassette d'expression selon la revendication 4, dans laquelle le gène d'intérêt code pour une protéine choisie parmi une protéine impliquée dans le développement de l'embryon et/ou de l'albumen, la croissance cellulaire, le métabolisme des sucres ou celui des acides gras, une protéine toxique, et une protéine inhibitrice de la transcription.

6. Cassette d'expression selon l'une quelconque des revendications 4 ou 5, dans laquelle le gène d'intérêt code pour une protéine choisie parmi la barnase ou l'isopentényltransferase.

7. Vecteur d'expression contenant une cassette d'expression selon l'une quelconque des revendications 4 à 6.

8. Cellule hôte transformée de plante Angiosperme, notamment de céréale, comprenant un vecteur selon la revendication 7.

9. Plante transgénique ou partie de plante transgénique, notamment fruit, semence, grain, pollen, générée à partir d'une cellule selon la revendication 8 et contenant dans son génome une cassette d'expression selon l'une quelconque des revendications 4 à 6.

10. Plante ou partie de plante selon la revendication 9, **caractérisée en ce qu'**il s'agit d'une céréale ou d'une plante oléagineuse, choisie notamment parmi le maïs, le blé, le colza, le tournesol, préférentiellement le maïs.

11. Plante transgénique hybride obtenue par croisement de plantes telles que définies dans l'une quelconque des revendications 9 ou 10 et contenant dans son génome une cassette d'expression selon l'une quelconque des revendications 4 à 6.

12. Procédé d'obtention d'une plante Angiosperme présentant des qualités agronomiques ou nutritionnelles améliorées, comprenant les étapes consistant à :
a) transformer au moins une cellule de plante Angiosperme par un vecteur selon la revendication 7 ; et
b) cultiver la cellule ainsi transformée de manière à générer une plante contenant dans son génome une cassette d'expression selon l'une quelconque des revendications 4 à 6.

13. Utilisation d'une cassette d'expression telle que définie dans l'une quelconque des revendications 4 à 6, pour l'obtention de plante Angiosperme transgénique présentant des qualités agronomiques ou nutritionnelles améliorées.

14. Utilisation selon la revendication 13, pour l'obtention d'une plante transgénique productrice de graines a teneurs en amidon ou en huile modifiées en comparaison avec une plante non transformée.

15. Utilisation de plante ou partie de plante, notamment semence, grain et fruit, telle que définie dans l'une quelconque des revendications 9 à 11, pour la préparation de produits dérivés, notamment de produits alimentaires.

## Patentansprüche

1. Isolierte Promoter-Nukleotidsequenz, die eine Expression von kodierenden Sequenzen ermöglicht, an die sie gebunden ist, wobei die Expression i) spezifisch ist für den Bereich des Nährgewebes, das den Keim in den Körnern von bedecktsamigen Pflanzen umgibt, und ii) insbesondere in den frühen Stadien der Entwicklung des Nährgewebes vorkommt, bestehend aus der SEQ ID N° 7 oder einer Sequenz, die zu mindestens 80 % zu SEQ ID N° 7 identisch ist.

2. Promoter-Nukleotidsequenz gemäß Anspruch 1, isoliert aus Getreide, insbesondere aus Mais.

3. Promoter-Nukleotidsequenz gemäß einem der Ansprüche 1 bis 2, umfassend des Weiteren ein cis-regulatorisches Element definiert durch das Motiv CTACACCA, vorzugsweise in Tandem Repeats.

4. Expressionskassette umfassend eine Promoter-Nukleotidsequenz gemäß einem der Ansprüche 1 bis 3, operabel gebunden an mindestens ein Gen von Interesse.

5. Expressionskassette gemäß Anspruch 4, wobei das Gen von Interesse für ein Protein kodiert, das ausgewählt ist aus einem Protein, das involviert ist in der Entwicklung des Keims und/oder des Nährgewebes, im Zellwachstum, im Zucker- oder Fettsäurestoffwechsel, einem toxischen Protein und einem Protein, das die Transkription inhibiert.

6. Expressionskassette gemäß einem der Ansprüche 4 oder 5, wobei das Gen von Interesse für ein Protein ausgewählt aus Barnase oder Isopentenyl-Transferase kodiert.

7. Expressionsvektor enthaltend eine Expressionskassette gemäß einem der Ansprüche 4 bis 6.

8. Bedecktsamige Pflanzen-Wirtszelle, insbesondere Getreide, umfassend einen Vektor gemäß Anspruch 7.

9. Transgene Pflanze oder Teil einer transgenen Pflanze, insbesondere Frucht, Samen, Korn, Blütenstaub, hergestellt aus einer Zelle gemäß Anspruch 8 und enthaltend eine Expressionskassette gemäß einem der Ansprüche 4 bis 6 in ihrem Genom.

10. Pflanze oder Pflanzenteil gemäß Anspruch 9, **gekennzeichnet dadurch, dass** es sich um ein Getreide oder eine Öl-haltige Pflanze handelt, ausgewählt insbesondere aus Mais, Weizen, Raps, Sonnenblume, vorzugsweise Mais.

11. Hybride transgene Pflanze erhalten durch Kreuzung von Pflanzen wie in einem der Ansprüche 9 oder 10 definiert und enthaltend eine Expressionskassette gemäß einem der Ansprüche 4 bis 6 in ihrem Genom.

12. Verfahren zur Herstellung einer bedecktsamigen Pflanze, die verbesserte landwirtschaftliche oder Nährwert-bezogene Eigenschaften aufweist, umfassend die Schritte bestehend aus:
a) Transformieren mindestens einer Zelle einer bedecktsamigen Pflanze durch einen Vektor gemäß Anspruch 7; und
b) Kultivieren der so transformierten Zelle, so dass eine Pflanze erzeugt wird, die eine Expressionskassette gemäß einem der Ansprüche 4 bis 6 in ihrem Genom enthält.

13. Verwendung einer Expressionskassette wie in einem der Ansprüche 4 bis 6 definiert, um eine transgene bedecktsamige Pflanze zu erhalten, die verbesserte landwirtschaftliche oder Nährwert-bezogene Eigenschaften aufweist.

14. Verwendung gemäß Anspruch 13, um eine transgene Pflanze zu erhalten, die, im Vergleich mit einer nicht-transformierten Pflanze, Körner mit einem modifizierten Gehalt an Stärke oder Öl produziert.

15. Verwendung einer Pflanze oder eines Pflanzenteils, insbesondere des Samens, des Korns und der Frucht, wie in einem der Ansprüche 9 bis 11 definiert, für die Herstellung von Folgeprodukten, insbesondere Nahrungsmittelprodukten.

## Claims

1. Isolated promoter nucleotide sequence allowing an expression of the coding sequences to which it is bound, said expression being i) specific to the region of the albumen surrounding the embryo in seeds of Angiosperms and ii) intervening in particular in the early stages of development of the albumen, consisting in SEQ ID N°7 or a sequence at least 80% identical to SEQ ID N°7.

2. Promoter nucleotide sequence according to claim 1, isolated from cereals, notably from maize.

3. Promoter nucleotide sequence according to any one of claims 1 to 2, further comprising a regulator element in *cis* defined by the pattern CTACACCA, preferably repeated in tandem.

4. Expression cassette comprising a promoter nucleotide sequence according to any one of claims 1 to 3, operatively bound to at least one gene of interest.

5. Expression cassette according to claim 4, in which the gene of interest codes for a protein is chosen from amongst a protein involved in the biological development of the embryo and/or of the albumen, the cell growth, the metabolism of sugars or of the fatty acids, a toxic protein, and a transcription inhibiting protein.

6. Expression cassette according to any one of claims 4 or 5, in which the gene of interest codes for a protein chosen from amongst barnase or isopentenyltransferase.

7. Expression vector containing an expression cassette according to any one of claims 4 to 6.

8. Transformed host cell of an Angiosperm plant, notably of a cereal, comprising an expression vector according to claim 7.

9. Transgenic plant or part of a transgenic plant, notably fruit, seed, grain, pollen, generated from a cell according to claim 8 and containing in its genome an expression cassette according to any one of claims 4 to 6.

10. Plant or part of a plant according to claim 9, **characterised in that** it is a cereal or an oily plant chosen notably amongst maize, wheat, rape, sunflower, preferably maize.

11. Hybrid transgenic plant obtained by crossing plants as defined in any one of claims 9 and 10 and containing in its genome an expression cassette according to any one of claims 4 to 6.

12. A method of obtaining an Angiosperm plant having improved agronomic or nutritional qualities, comprising the steps consisting of:
a) transforming at least one Angiosperm plant cell by means of an expression vector according to claim 7; and
b) cultivating the cell thus transformed so as to generate a plant containing in its genome an expression cassette according to any one of claims 4 to 6.

13. Use of an expression cassette as defined in any one of claims 4 to 6, for obtaining a transgenic Angiosperm plant exhibiting improved agronomic or nutritional qualities.

14. Use according to claim 13, for obtaining a transgenic plant producing seeds with starch or oil contents modified compared with a non-transformed plant.

15. Use of a plant or part of a plant, notably seed, grain and fruit, as defined is any one of claims 9 to 11, for the preparation of derived products, notably food products.


### LISTE DE SEQUENCES

<110> Biogemma
<120> Promoteurs spécifiques de l'albumen des graines de végétaux
<130> BFF 99/496ext
<140>
   <141>
<160> 21
<170> PatentIn Ver. 2.1
<210> 1
   <211> 531
   <212> ADN
   <213> Zea mays
<400> 1
   gatcattaag gactaagcag tctttttccc tttcggcttg catcatcttt agtcttcatc 60
   actattataa gccgaagcta ttaccccttg gctatagctt cggtgttcat ctttattatc 120
   ttcggactat gtcttcacct tgtatacctt tgtcttgggg gaaaaccttc atcctgaagc 180
   cgaagctccc tgtaataatt catatcatgc taaaaataaa tggtcagtcc tgtttttgag 240
   gaccttcgga agaggaaggc cccccaacaa gacgattaac tagtattgtc tcactgcatt 300
   gtttttttgg cacttcatca ataatgcctc aatagcatac ttcattttag gaactttatt 360
   aaaactgtct taaaataggg ccaagtcata aattcattca aagtgactct tcatttctta 420
   cttcctatct ttggtggttt tgtatatata tatgttcatg gttgagtgat gttcctacac 480
   cactacacca cacgttagat atatatacag aaaatagctt cactatctag a          531
<210> 2
   <211> 2493
   <212> ADN
   <213> Zea mays
<400> 2
   aagcttttcc ggtgatgaag cacctgtaat acttaacagc atgctgaaaa caaatagtta 60
   gctgtgtttt tgaggacctt cggaagatga aggcccccaa cacatcccat gcatcaagtc 120
   cccatgactt gcaaaaaagc aaattttatc aaaatttctc ataaaacact tgaaaacatt 180
   tctctttttg aaaagtgtag agcactagca actgtctact aaaaaggttc ccaaatttct 240
   gggtataaca atcgcatggt aaataacaca aaggaaatcc tactaagagc agtaatttgg 300
   ctaaaacaat agtgagcatt ttaatgtaat agggaatagg agcatgcaat acttgtgttc 360
   tttcagggtt ttgatgtcct caaaagtgtg cccccctggg gcagttgcaa cactcaaaat 420
   ctactcgtat acataaagaa acatgggcac aaaataagaa acaatactca aattatgaaa 480
   aaggttcaaa tggtcctata attattgtag acattttaga atttatttta gaccaaaacc 540
   atttaaattt ggtttaaaat gagttagata ttaatattta ttcagtttat agttatttgg 600
   gacatttatt tacttaacta taacttctag ggttttaaaa gtaaattttg ggtccctagt 660
   tggaactagc tcagattgct ggttgatttc cataaaagtc gaggttcctt tagcaaaaat 720
   ccacggtgaa caagggggag ataggtgttg accgatatct ctaaattttg atcgttggac 780
   ggcacatgga tgtctcagat taaatggtgg atgtgcaagc gacgcgcaca cgatggagga 840
   atggcttcac gacggtgggc tactagagct ggctacgtca accaatggag ggctcggtca 900
   aggtcaaaat ttgttgccaa gccactgtgg ctcacgatga gtcgattgag cacatatcaa 960
   ggtcgagggt caaccagagg ggcaagatcg atggtgcagt ggtgttctcg atggaagggg 1020
   aaacttcggt gagcaattca agatttccta tcatgtgacc gggtcaggga atgggcgcat 1080
   ggggtttggt accttctggt gcacatcatg ttgctgtatc gatgtcaagg gagcattagg 1140
   gttcacgagt cagcgatgac gggcatggtg ggacttgtgt caccatggtt cgatcaacta 1200
   gggacgatag agctctatga agtttcacaa cttcctcaca ctctagggat catggtgaca 1260
   aaggtgggga ggacggggcg tctctagtga gggtggaatg cagttctgtc acgtgggaat 1320
   agtggcggca tcgcttgtaa tgaataaaag gtgcttgggt ggctgggaag tgcaatatga 1380
   gggaagtagt tggtgcgggg atgttccttt tataagggag caccattgat taatggaaga 1440
   caatgacaca aagggtggtg cgacagttta aagctcgaat gctgctaggg gtgctcaagg 1500
   ttaaaagatc aggcatcagg gaggaaaggc agggataaaa tttctttact ccagttgtgg 1560
   ggtgatgggg acaagggtag tgctcaagca agggagggcg agttcagcgc agagatgcct 1620
   gttgtgacac atgggggggg gggaattgga ggttggggtt gaccaggtga cgttatggcg 1680
   tgacccagag aagagaccca ctgatgggga aaaaaggtgc caacaggtgg ggaccaaggt 1740
   gtcagtgact caccgtgaca tgttattgga aagttacgtc cggaatggtt tgggcctgag 1800
   tgatctaggc tggctcgggc actgtgctga tcctttaatt tctccattcc caatttaagt 1860
   tgaattttta attcaaatca aatgactcca aatctctcca aaattaccaa aatatagaat 1920
   atttagatga atatgttggt ggagtttggg ctccgctttt ggttagtatg tttgtataaa 1980
   aataatttct ctccttttgt cacttccaat attgacttaa atttttatgt agcaatgcca 2040
   acttttttta gtagtgtgcc acttatagca caaaaactat atccattttc taatagtcct 2100
   tgaaatccac attctatttt tagccattct tcaaaattgg cacaaaacta ggaaaattta 2160
   atacattctt gccataacat attctagtgc aaatgttaac tagattgctc aatattagca 2220
   aacttctttt gtaagattca ttaatattgc tacattgcat acttttttag aagttcatca 2280
   ataatgcctc attagcatac ttcattttag gaacttgatt aaaaccgcct taaaatagag 2340
   ccaagtgacg gatccattta aaggtgattc ttaatttctt acttcctatc tttggtggct 2400
   tatgtttata tatgtgtggg tggttgaatg atgttcctac accactacac cacacgttgg 2460
   acatatatat ggaaaatagc ttcacagtct aga 2493
<210> 3
   <211> 1708
   <212> ADN
   <213> Zea mays
<400> 3
   aagcttagaa attttaaaaa aagccaggca agcgttggtg tgcaaagagc taaaaattag 60
   gaagacaaga gaacacggca agaaagcatg ctaaatgtgc tcgcggtgcg ttcttattta 120
   tacgctcaat acgttgcaag tggtagggcc ccacttgtca ttgactattg ctattctagc 180
   aaagggaagg tgtttttcgg accttcggct taaggccttc gtccatatcg caatctgaat 240
   ttatcattct aacaaattaa tattgtgagg ggctactgtt gggggccttc gacttccgaa 300
   ggtcctcaaa aactggttta acagtgtttc tggagtataa tgcataaaca ggtatcttcg 360
   ggtttggatc agaactacaa catgaagagg cacaaagaac acgaaggttg gcgcagagcc 420
   gaagctcacg tgtaggagag cttcggcacg acagcagaaa aagggaaccg acttaaaagg 480
   aaaggctatt cagacctcga tggatttcta taggtcatta gcaaatgtaa agggcatgaa 540
   tgtaatttta catgggctgt gtccttgcct ataaatagat gaacagtact ctcgtactgt 600
   tcacgctgac ttggcattcg ctttttgcat cacgcttgta cccttgcttt ccttcaaacc 660
   gaaggtacat ctataatttg ttattgtgtt attgtggata tggtaatgca aataaaaata 720
   agttgatgat aatgtttata ttatttttcg tatttcatat atgaattctt cctcatcatt 780
   tattgtgctt acgaaggttt ttccttcaaa atctttgtcc ggaattcatt atatccgaag 840
   ggaaataatg tctcgaagga cgaaggactt tgatatttaa cacttttcat gttgccttgt 900
   tcttgactct tagcatttga gaacaagtcc ccaacagctc ctaagctctt ctttgaagaa 960
   acaactacta gatgaagttt ctccaaaagt acgtccattg aatggagtaa agagtcattt 1020
   gacctctcgg aataaaatta aaatgagaat aagtaagaat aaaacacctc tattatcaaa 1080
   tctaggccat acaaacattg ggtattacta aaaaatagct aatgccatct ttcaacattt 1140
   ggaagttaaa accaaccaat cctcactcat tcccaagaaa tattggatca tatttaacat 1200
   tttgtgtcac ttacaaaaat ggcttaatct tttatgcggc aatgccaacc ttttttagca 1260
   gggtgccact tgtaacatga aaactataac tattttcaaa tagtaccttg aaattcgcat 1320
   tctattttta tgcattcttc aaaattgaca caaattaaac taggagaatt caatacattc 1380
   ttgccataac atattctaat gcaaatatta agtagattgc tcaacatcgg tacacatctt 1440
   ttggacgatt aattagtatt gtctcactac attctttgtt ttagcagttc atcaataatg 1500
   cctcaatagc atacttcatt ttaggaactt tatgaaaatt gtcttaaaat agggccaagt 1560
   cacaaatcca cttcaaaggt gactcttcat ttcttacttc ctatctttgc ttgtttttgt 1620
   atatatatgt gtggatggtt gagtgatgtt cctacaccac tacaccacac cttagacaca 1680
   tatatggaaa atagcttcac tgtctaga                                    1708
<210> 4
   <211> 1232
   <212> ADN
   <213> Zea mays
<400> 4
   tagttcatgc aaaagtagtg agtgtttata cacctatgcc aatgaataac ctctaactac 60
   cctcatgtac tggcctggtg tttggtaaac atgaagcaat aatttcctct tatcaaaata 120
   cgggtctcaa gctgcatgtg aaaaataata aatatttttt tgaagtgaat gaaaaatgat 180
   aaatatgaaa cagtaaatct ttccgttgaa aaagtacatc tctattatta acgataacct 240
   atatatcaat ctacaatgcg ctcatctgca tctcgatgca tactttcatc attttatgaa 300
   tgtactttaa tgataagaag gattagaatg ttcttgtttt cctcttattc ttaccttttt 360
   caaaattatc agtttccaat gtctgaatat gcaatgcatt ataaacccta gtcagcatat 420
   atcaagtcga tatataatgc tatatgttta agaactggtg ctgagtatgt ctactcaaca 480
   tattttttag ctattggatc gagcagttta gtaaaggtaa actacattta tctatcttca 540
   agttgtattt tcccaccctt aaattatgaa agggagtaac gctccactcc aactgttgaa 600
   agggaacaaa tttggtctcc ggactgattt cattggtggt tctctatttt ttaaaacaac 660
   aaaaaaacat atttgttctc tgaaaattga taattaatta atcataaatt aggaaaaaaa 720
   ctatatgaaa ctagttatag ttttcttcta aaattattgt ctgtctgttg gtgctctagt 780
   tatagagtta taacatgaaa actatagcca ttttcaaata gtgccttgaa attcattttt 840
   gtttagccat tcttcaaaat tgccaccaaa ccaggagaat ttattaataa attcacgcca 900
   taacatattc tagtccaaat gttaagtgga ttgctcaata tcattatact tcttttggac 960
   gattcattag tactgccttg ttgcatactt tgttttagca gttcatcaat aatgcttcac 1020
   tagcataatt catgttagga acttgattaa aactgcctta agaacatggc aaagtgataa 1080
   atccacttca aaggcaatta ttaatttctt acttcctatc tttggtggtt tttgtatata 1140
   tgtgtgtggg tggttgagtg atgcacacat tttcctacac cactacatca caccttggac 1200
   atatatgtga aaaaatagct tcgcagtcta ga                               1232
<210> 5
   <211> 499
   <212> ADN
   <213> Zea mays
<400> 5
   ttttgtcact tccaatattg acttaaattt ttatgtagca atgccaactt tttttagtag 60
   tgtgccactt atagcacaaa aactatatcc attttctaat agtccttgaa atccacattc 120
   tatttttagc cattcttcaa aattggcaca aaactaggaa aatttaatac attcttgcca 180
   taacatattc tagtgcaaat gttaactaga ttgctcaata ttagcaaact tcttttgtaa 240
   gattcattaa tattgctaca ttgcatactt ttttagaagt tcatcaataa tgcctcatta 300
   gcatacttca ttttaggaac ttgattaaaa ccgccttaaa atagagccaa gtgacggatc 360
   catttaaagg tgattcttaa tttcttactt cctatctttg gtggcttatg tttatatatg 420
   tgtgggtggt tgaatgatgt tcctacacca ctacaccaca cgttggacat atatatggaa 480
   aatagcttca cagtctaga                                              499
<210> 6
   <211> 507
   <212> ADN
   <213> Zea mays
<400> 6
   ttgtgtcact tacaaaaatg gcttaatctt ttatgcggca atgccaacct tttttagcag 60
   ggtgccactt gtaacatgaa aactataact attttcaaat agtaccttga aattcgcatt 120
   ctatttttat gcattcttca aaattgacac aaattaaact aggagaattc aatacattct 180
   tgccataaca tattctaatg caaatattaa gtagattgct caacatcggt acacatcttt 240
   tggacgatta attagtattg tctcactaca ttctttgttt tagcagttca tcaataatgc 300
   ctcaatagca tacttcattt taggaacttt atgaaaattg tcttaaaata gggccaagtc 360
   acaaatccac ttcaaaggtg actcttcatt tcttacttcc tatctttgct tgtttttgta 420
   tatatatgtg tggatggttg agtgatgttc ctacaccact acaccacacc ttagacacat 480
   atatggaaaa tagcttcact gtctaga                                    507
<210> 7
   <211> 265
   <212> ADN
   <213> Zea mays
<400> 7
   namgattmay tartattgyy wcaytrcatw snttnttttr gmasttcatc aataatgcct 60
   cawtagcata cttcatttta ggaacttkat kaaaayygyc ttaaaatagr gccaagtsay 120
   rratycantt yaaagntgay tcttmatttc ttacttccta tctttgstkg yttwngtwta 180
   tatatrtgtk srtggttgar tgatgttcct acaccactac accacacstt rgayayatat 240
   ayrgaaaata gcttcacwrt ctaga                                      265
<210> 8
   <211> 28
   <212> ADN
   <213> Séquence artificielle
<220>
   <223> Description de la séquence artificielle:oligonucléotide
<400> 8
   ggggtctaga ctgtgaagct attttcca 28
<210> 9
   <211> 30
   <212> ADN
   <213> Séquence artificielle
<220>
   <223> Description de la séquence artificielle:oligonucléotide
<400> 9
   ggggaagctt tacattcttg ccataacata 30
<210> 10
   <211> 30
   <212> ADN
   <213> Séquence artificielle
<220>
   <223> Description de la séquence artificielle:oligonucléotide
<400> 10
   ggggaagctt ttcatcaata atgcctcatt 30
<210> 11
   <211> 30
   <212> ADN
   <213> Séquence artificielle
<220>
   <223> Description de la séquence artificielle:oligonucléotide
<400> 11
   ggggaagctt taatttctta cttcctatct 30
<210> 12
   <211> 27
   <212> ADN
   <213> Séquence artificielle
<220>
   <223> Description de la séquence artificielle:oligonucléotide
<400> 12
   ggccagtcga caaagcggcc gcatgca 27
<210> 13
   <211> 19
   <212> ADN
   <213> Séquence artificielle
<220>
   <223> Description de la séquence artificielle:oligonucléotide
<400> 13
   tcagctgttt cgccggcgt 19
<210> 14
   <211> 14
   <212> ADN
   <213> Séquence artificielle
<220>
   <223> Description de la séquence artificielle:oligonucléotide
<400> 14
   tcgactgcag ccca 14
<210> 15
   <211> 14
   <212> ADN
   <213> Séquence artificielle
<220>
   <223> Description de la séquence artificielle:oligonucléotide
<400> 15
   gacgtcgggt tcga 14
<210> 16
   <211> 16
   <212> ADN
   <213> Séquence artificielle
<220>
   <223> Description de la séquence artificielle:oligonucléotide
<400> 16
   ctagacccga attcgc 16
<210> 17
   <211> 16
   <212> ADN
   <213> Séquence artificielle
<220>
   <223> Description de la séquence artificielle:oligonucléotide
<400> 17
   tgggcttaag cgccgg 16
<210> 18
   <211> 15
   <212> ADN
   <213> Séquence artificielle
<220>
   <223> Description de la séquence artificielle:oligonucléotide
<400> 18
   gatccactag tcccg 15
<210> 19
   <211> 15
   <212> ADN
   <213> Séquence artificielle
<220>
   <223> Description de la séquence artificielle:oligonucléotide
<400> 19
   aattcgggac tagtg 15
<210> 20
   <211> 17
   <212> ADN
   <213> Séquence artificielle
<220>
   <223> Description de la séquence artificielle:oligonucléotide
<400> 20
   aagctttttg cggccgc 17
<210> 21
   <211> 25
   <212> ADN
   <213> Séquence artificielle
<220>
   <223> Description de la séquence artificielle:oligonucléotide
<400> 21
   tcgagcggcc gcaaaaagct tagct 25